(19) Europäisches Patentamt
European Patent Office
Office européen des brevets

(11) EP 2 054 463 B1

(12) **EUROPÄISCHE PATENTSCHRIFT**

(45) Veröffentlichungstag und Bekanntmachung des Hinweises auf die Patenterteilung:
**16.02.2011 Patentblatt 2011/07**

(21) Anmeldenummer: **07788229.8**

(22) Anmeldetag: **03.08.2007**

(51) Int Cl.:
***C08J 3/14*** *(2006.01)*

(86) Internationale Anmeldenummer:
**PCT/EP2007/058098**

(87) Internationale Veröffentlichungsnummer:
**WO 2008/025640 (06.03.2008 Gazette 2008/10)**

(54) **VERFAHREN ZUR HERSTELLUNG VON MIKROPARTIKELN**

METHOD FOR PRODUCING MICROPARTICLES

PROCÉDÉ DESTINÉ À PRODUIRE DES MICROPARTICULES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.08.2006 DE 102006040123**

(43) Veröffentlichungstag der Anmeldung:
**06.05.2009 Patentblatt 2009/19**

(73) Patentinhaber: **Evonik Röhm GmbH**
**64293 Darmstadt (DE)**

(72) Erfinder:
- **SEILER, Matthias**
  **64347 Griesheim (DE)**
- **IRFAN, Muhammad**
  **Friederich-Alexander-Universität Erlangen-Nürnberg**
  **91058 Erlangen (DE)**

(56) Entgegenhaltungen:
**WO-A-2007/048464**
**DE-A1- 3 916 020**
**US-A- 5 665 428**
**US-A1- 2004 109 894**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 054 463 B1

## Beschreibung

Die vorliegende Erfindung betrifft Verfahren zur Herstellung von Mikropartikeln und Anlagen zur Durchführung dieser Verfahren.

Mikropartikel sind an sich seit langem bekannt. Üblicherweise werden diese Partikel mit verschiedenen Methoden, insbesondere Koazervation, RESS-, GAS und/oder PGSS-Verfahren sowie Prozessen unter Verwendung von Koaxialdüsen, Sprühtrocknung oder Wirbelschichtcoating hergestellt.

Beim RESS Verfahren (Rapid Expansion of Supercritical Solution) wird ein Gemisch umfassend ein überkritisches Fluid und eine darin gelöste Substanz plötzlich entspannt. Wesentlich ist hierbei die Löslichkeit der Substanz in dem überkritischen Fluid, welches die Anwendbarkeit dieses Verfahrens begrenzt. Bei einer geringen Löslichkeit der Substanz werden sehr große Gasmengen beim Entspannen der Mischung freigesetzt. Eine nähere Erläuterung dieses Verfahrens mit weiteren Nachweisen ist in Gamse et al., Chemie Ingenieur Technik 77 (2005), No. 6, Seiten 669 bis 679 dargestellt.

Die Problematik der geringen Löslichkeit vieler Substanzen in überkritischen Gasen wird durch das GAS-Verfahren (Gas Anti Solvent) umgangen. Bei GAS-Verfahren wird zunächst eine Lösung der Substanz bzw. Substanzen hergestellt, die in die Partikelform überführt werden soll. Zu dieser verdünnten Lösung wird ein gasförmiges oder überkritisches Fällungsmittel hinzugegeben. Hierdurch fällt die Lösungskapazität des Lösungsmittels ab, so dass sich durch die schlagartige Übersättigung feinste Partikel bilden. Diese Verfahren wurden insbesondere von Gamse et al., Chemie Ingenieur Technik 77 (2005), No. 6, Seiten 669 bis 679 und Tom, J.W.; Lim, G.B.; Debendetti, P.G.; Prod'homme, R.K.Supercritical Fluid Engineering Science, Washington DC 1993: Brennecke, J.F.; Kiran, E.; American Chemical Society: 1993, dargelegt.

Bei den PGSS-Verfahren wird ein komprimiertes Gas zu einer Schmelze hinzugefügt. Diese mit Gas gesättigte Schmelze wird anschließend über eine Düse auf Atmosphärendruck entspannt. Durch die Verdampfung des komprimierten Gases kann eine Abkühlung des Systems auftreten. Falls die Temperatur unterhalb der Verfestigungstemperatur der Schmelze fällt, werden feste Partikel gebildet. Die PGSS-Verfahren wurden unter anderem von Gamse et al., Chemie Ingenieur Technik 77 (2005), No. 6, Seiten 669 bis 679; Pérez de Diego, Y. Production of Controlled Drug Delivery Microparticles using Supercritical CO2 2004; und Shariati, A.; Peters, C.J. Recent developments in particle design using supercritical fluids. Current Opinion in Solid State & Materials Science 2003, 7 (4-5), 371-383 beschrieben.

Die zuvor beschriebenen Verfahren, die mit Hochdruck arbeiten, insbesondere die RESS-, GAS und/oder PGSS-Verfahren benötigen oft hohe Mengen an Energie. Des Weiteren werden vielfach teuere und für bestimmte Zwecke unerwünschte Lösungsmittel eingesetzt. Darüber hinaus treten vielfach hohe Prozesstemperaturen auf, die wiederum mit einem hohen Energiebedarf verbunden sind. Aufgrund der Druckbedingungen und Prozesstemperaturen können chemisch instabile Verbindungen vielfach nicht mit diesen Verfahren verarbeitet werden. Darüber hinaus sind diese Verfahren in der Durchführung mitunter unflexibel und vielfach auf eine bestimmte Partikelgröße beschränkt. Ein Hauptnachteil besteht in dem hohen apparativen Aufwand, der zur Durchführung notwendig ist. Die eingesetzten Anlagen sind häufig sehr wartungsintensiv und verursachen hohe Investitionskosten. Darüber hinaus erfordern die hohen Prozessdrücke zusätzliche Sicherheitsvorkehrungen.

Bei der Koazervation werden die Partikel durch Fällung aus einer Lösung von Polymer und niedermolekularer Substanz gebildet.

Man unterscheidet im Allgemeinen zwischen einfacher und komplexer Koazervation, sowie zwischen wässriger und organischer Phasentrennung (R. Arshady, "Microspheres and microcapsules - A survey of manufacturing techniques, Part III: Coacervation", Polymer Engineering and Science 30 (1990) 905ff). Bei der einfachen Koazervation kommt eine kolloidale Komponente, z. B. Gelatine, und bei der komplexen Koazervation zwei gegensätzlich geladene kolloidale Komponenten, z.B. Gelatine und Gum Arabicum, zum Einsatz. Das Prinzip der Koazervation besteht darin, dass z.B. eine wässrige Gelatinelösung durch Zugabe von Ethanol in ein Zweiphasen-System überführt wird, das aus einer Gelatine-reichen (Koazervat) und eine Gelatine-armen Phase besteht.

Dieses Verfahren wurde unter anderem von R. Arshady, "Microspheres and microcapsules - A survey of manufacturing techniques, Part III: Coacervation", Polymer Engineering and Science 30 (1990) 905ff. ; Jain, R.A. The manufacturing techniques of various drug loaded biodegradable poly(lactide-co-glycolide) (PLGA) devices. Biomaterials 2000, 21 (23), 2475-2490; Jung, J.; Perrut, M. Particle design using supercritical fluids: Literature and patent survey. Journal of Supercritical Fluids 2001, 20 (3), 179-219; und Subramaniam, B.; Rajewski, R.A.; Snavely, K. Pharmaceutical processing with supercritical carbon dioxide. Journal of Pharmaceutical Sciences 1997, 86 (8), 885- 890 beschrieben. Bei diesem Verfahren ist ebenfalls die Verwendung von Lösungsmitteln, die aufwendig abgetrennt werden müssen, von Nachteil. Darüber hinaus ist der Fällungsprozess kritisch, so dass Schwierigkeiten im Hinblick auf die gewünschten Partikelgrößen und die gewünschten Partikelgrößenverteilungen auftreten können.

Darüber hinaus können Mikropartikel, die im Allgemeinen Wachse als Verkapselungsmaterial aufweisen, durch so genannte Schmelzdispersionsverfahren erhalten werden. Hierbei wird ein Wirkstoff in einer Wachsschmelze dispergiert oder gelöst. Die erhaltene Schmelze wird anschließend in einer externen Phase emulgiert und abgekühlt. Derartige Verfahren sind unter anderem in C. M. Adeyeye, J. C. Price, Pharmaceutical

Research, 1991, Vol. 8, No. 11, 1377-1383; A. Paradkar et al., AAPS PharmSciTech 2003, 4 (4) Article 65; A. Raziel, "Wax Microemboli Tailored for Therapeutic Embolization" AJR 134, February 1980, 404-405; S. Benita et al., Journal of Pharmaceutical Sciences, Vol. 75, No. 9, September 1986, 847-851; N. Mani, Drug Development and Industrial Pharmacy 2004, Vol. 30, No. 1, 83-93; und N. Mani et al, J. Microencapsulation 2004, Vol. 21, No. 2, 125-135 dargelegt. Allerdings finden sich hierin keine Hinweise auf die Verwendung von polymeren Matrixmaterialien.

Ähnliche Verfahren, die unter Verwendung von Polymeren als Matrixphase arbeiten, werden in DE 39 16 020; WO 97/15389 und E. Mathiowitz, R. Langer, Journal of Controlled Release, 1987, Vol. 5, 13-22 beschrieben. Allerdings werden vielfach sehr breite Partikelverteilungen erhalten, wobei die Teilchengröße vielfach nur sehr schwer auf ein vorgegebenes Ziel eingestellt werden kann. So wird insbesondere in DE 39 16 020 dargelegt, dass das Verfestigen der Polymerschmelze durch Eingießen in kaltes Wasser zu einer sehr breiten Partikelgrößenverteilung führt. Gemäß WO 97/15389 werden unterschiedliche kontinuierliche Phasen eingesetzt, um die Partikel zu erhalten, wobei wässrige Phasen vielfach zur Bildung von Aggregaten und einer schlechten Partikelform führen. Gemäß der von Mathiowitz dargelegten Lehre wird eine hydrophobe Phase eingesetzt, wobei insbesondere Cyclohexan zur Isolierung der erhaltenen Partikel eingesetzt wird. Kontinuierliche Verfahren werden in diesen Druckschriften nicht beschrieben. Darüber hinaus finden sich in diesen Druckschriften keine Hinweise auf Möglichkeiten die Partikelgrößenverteilung zu minimieren oder die Größe der Partikel auf einen vorgegebenen Zielbereich einzustellen.

Die Sprühtrocknung ist ein kontinuierlich durchführbares Verfahren zur Trocknung von Lösungen, Suspensionen oder pastösen Massen. Dieses Verfahren ist weithin bekannt, wobei Anlagen zur Durchführung des Verfahrens kommerziell erhältlich sind. Im Allgemeinen wird Mittels einer Düse (durch Flüssigkeitsdruck oder Pressluft bzw. Inertgas betrieben) oder rotierenden Sprühscheiben (4000-50000 U/min) das zu trocknende Gut in einen Heißluftstrom (Temperaturen je nach Apparatur bis zu 220°C) eingebracht, der es in Bruchteilen einer Sekunde zu einem feinen Pulver trocknet. Die Heißluft kann in Richtung mit dem Sprühstrahl oder gegen den Sprühstrahl strömen (Gleichstrom-, Gegenstromverfahren), je nach Bauart oder Verwendungszweck. Die Sprüheinrichtung befindet sich am oberen Teil eines Sprühturms, das anfallende Trockengut wird meist durch einen Zyklonabscheider vom Luftstrom getrennt und kann dort entnommen werden. Problematisch an diesem Verfahren ist neben dem Energieverbrauch ebenfalls die Erzielung von bestimmten Partikelgrößen und Partikelgrößenverteilungen, da die Partikel während des Herstellungsverfahrens zur Aggregation neigen. Des Weiteren wird vielfach nur eine unzureichende Schutzwirkung erzielt, da die erhaltene Oberfläche sehr rau ist. Ferner sind die Temperaturen während des Sprühens sehr hoch, da die Viskosität der Zusammensetzung relativ gering sein muss, um kleine Partikel zu erhalten. Daher eignet sich dieses Verfahren nicht zur Verarbeitung von temperaturempfindlichen Substanzen. Die Zugabe von Lösungsmitteln zur Verringerung der Viskosität führt wiederum zu den zuvor genannten Problemen, wobei eine Explosionsgefahr hinzukommt, falls organische Lösungsmittel eingesetzt werden.

Darüber hinaus sind Verfahren zur Herstellung von Mikropartikeln bekannt, bei denen zunächst eine Lösung aus zwei oder mehr Substanzen, beispielsweise einem Polymer und einer zu verkapselnden Substanz hergestellt wird. Anschließend wird das Lösungsmittel verdampft. In einem nachfolgenden Schritt wird die getrocknete Zusammensetzung zu Mikropartikeln vermahlen (vgl. WO 05/072702, WO 04064752, DE 10061932, WO 00/72830, EP 914095, WO 97/42940 und Gamse et al., Chemie Ingenieur Technik 77 (2005), No. 6, Seiten 669 bis 679). Nachteilig an diesen Verfahren ist insbesondere die Erzielung von bestimmten Partikelgrößen und Partikelgrößenverteilungen. Des Weiteren wird vielfach nur eine unzureichende Schutzwirkung erzielt, da unregelmäßige Partikel mit einer rauen Oberfläche erhalten werden, wobei vielfach keine geschlossene Hülle um den Wirkstoff gebildet wird. Des Weiteren neigen viele Partikel zum Agglomerieren, wobei diese Neigung durch elektrostatische Aufladung während des Zerkleinerns verstärkt wird. Eine Zugabe von unerwünscht hohen Additivmengen, um diese Agglomeration zu vermeiden, ist im Allgemeinen erforderlich. Weiterhin erfordert das Mahlen eine hohe Energie, wobei das Mahlwerk an sich sehr heiß werden kann, so dass die Partikel partiell schmelzen und agglomerieren können. Hierbei können empfindliche chemische Substanzen abgebaut werden. Daher können instabile Substanzen wie Vitamine, Aromen etc. mit Mahlverfahren nur schwer verkapselt oder formuliert werden. Des Weiteren werden aus den zuvor genannten Gründen im Allgemeinen Partikel erhalten, die ein verbesserungsbedürftiges Freisetzungsverhalten der zu verkapselnden Substanz aufweisen.

In Anbetracht des Standes der Technik ist es nun Aufgabe der vorliegenden Erfindung Verfahren zur Herstellung von Mikropartikeln zur Verfügung zu stellen, die besonders einfach, kostengünstig und energiesparend durchgeführt werden können. Ein besonderes Problem bestand insbesondere darin ein Verfahren zur Herstellung von Mikropartikeln zu schaffen, das ohne hohen apparativen Aufwand und ohne die Verwendung von gesundheitlich bedenklichen Lösungsmitteln ausgeführt werden kann. Eine weitere Aufgabe kann darin gesehen werden, ein Verfahren zur Herstellung von Mikropartikeln anzugeben, das insbesondere mit empfindlichen Substanzen durchgeführt werden kann, ohne dass die Substanzen chemisch verändert werden. Ferner sollten die durch erfindungsgemäße Verfahren erhältlichen Mikropartikel eine hohe Haltbarkeit aufweisen, wobei insbesondere die zu verkapselnden Substanzen über einen langen Zeitraum gelagert werden können sollten, ohne dass eine chemische Veränderung auftritt. Des Weiteren sollten die erhaltenen Mikropartikel eine hohe Scherstabilität sowie ein vorteilhaftes Freisetzungsprofil aufweisen. Hierbei sollte die zu verkapselnde Substanz, je nach Anwendung, über einen besonders langen Zeitraum oder innerhalb eines kurzen Zeitraums freigesetzt werden können, wobei die Freisetzung durch externe oder interne Auslösemechanismen gesteuert werden können sollte. Weiterhin war es eine Aufgabe der vorliegenden Erfindung, ein Verfahren bereitzustellen, durch das besonders einheitliche Mikropartikel mit einer vorgegebenen Partikelgröße und Partikelgrößenverteilung erhalten werden können.

Gelöst werden diese sowie weitere nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen der erfindungsgemäßen Verfahren werden in Unteransprüchen unter Schutz gestellt. Darüber hinaus sind Anlagen zur Durchführung des erfindungsgemäßen Verfahrens Gegenstand dieser Anmeldung, wobei die Ansprüche 9 bis 11 eine Lösung der zugrunde liegenden Aufgabe darlegen.

Gegenstand der vorliegenden Erfindung ist ein Verfahren zur Herstellung von Mikropartikeln umfassend die Schritte

a) Herstellen einer Polymerschmelze umfassend mindestens ein Trägerpolymer und mindestens eine zu verkapselnde Substanz,

b) Einleiten der Polymerschmelze in eine zweite flüssige Phase, in der das Trägerpolymer schwer löslich ist, wobei die zweite flüssige Phase eine Verfestigungstemperatur aufweist, die unterhalb der Verfestigungstemperatur des Trägerpolymers liegt,

c) Dispergieren der Polymerschmelze in der zweiten flüssigen Phase bei einer Temperatur, die größer oder gleich der Verfestigungstemperatur des Trägerpolymers ist und

d) Verfestigen der in der zweiten flüssigen Phase dispergierten Polymerschmelze durch Abkühlen der zweiten flüssigen Phase auf eine Temperatur unterhalb der Verfestigungstemperatur des Trägerpolymers durch Zuleiten einer Kühlflüssigkeit in einem Mischventil.

Durch die erfindungsgemäßen Maßnahmen wird ein Verfahren zur Herstellung von Mikropartikeln zur Verfügung gestellt, das besonders einfach, kostengünstig und energiesparend durchgeführt werden kann.

Zugleich lassen sich durch die erfindungsgemäßen Verfahren eine Reihe weiterer Vorteile erzielen. Hierzu gehört unter anderem, dass durch das erfindungsgemäße Verfahren die Herstellung von Mikropartikeln ohne hohen apparativen Aufwand und ohne die Verwendung von gesundheitlich bedenklichen Lösungsmitteln gelingt. Durch das erfindungsgemäße Verfahren können insbesondere besonders einheitliche Mikropartikel mit einer vorgegebenen Partikelgröße und Partikelgrößenverteilung erhalten werden. Hierbei ist das Verfahren besonders flexibel. So können mit einer Anlage sowohl kleine als auch große Partikel mit einer jeweils relativ engen Partikelgrößenverteilung hergestellt werden. Ferner können Mikropartikel gebildet werden, die pulverförmige und/oder flüssige zu verkapselnde Substanzen aufweisen. Des Weiteren können die zu verkapselnden Substanzen auch im Trägerpolymer löslich sein oder ein Lösungsmittel für das Trägerpolymer darstellen. Weiterhin können die zu verkapselnden Substanzen in Wasser löslich oder unlöslich sein. Darüber hinaus kann das Verfahren bei relativ geringen Temperaturen durchgeführt werden, so dass temperatursensitive niedermolekulare Substanzen verkapselt und das Verfahren mit einem geringen Energiebedarf ausgeführt werden kann. Darüber hinaus kann das Verfahren der vorliegenden Erfindung mit einem hohen Durchsatz durchgeführt werden, so dass in kurzer Zeit große Mengen an Mikropartikeln gebildet werden können. Durch das vorliegende Verfahren können die Mikropartikel kontinuierlich gebildet werden. Des Weiteren können auch Mikropartikel erhalten werden, die empfindliche Substanzen aufweisen. Ferner kann das Verfahren ohne große Mengen an gesundheitlich bedenklichen Lösungsmitteln ausgeführt werden, die abgetrennt oder aufbereitet werden müssen. Die durch das vorliegende Verfahren erhältlichen Mikropartikel können ein hervorragendes Freisetzungsprofil der zu verkapselnden Substanz aufweisen, wobei sowohl eine Freisetzung über einen besonders langen Zeitraum als auch eine kurzzeitige Freisetzung nach Initiierung eines Freisetzungsmechanismus realisiert werden können. Die Mikropartikel können empfindliche Substanzen über einen besonders langen Zeitraum sicher lagern, wobei beispielsweise oxidationsempfindliche Substanzen vor einer oxidativen Zersetzung geschützt werden können. Darüber hinaus können die erfindungsgemäß erhältlichen Mikropartikel eine hohe Scherstabilität aufweisen. Hierdurch können die erfindungsgemäß erhältlichen Mikropartikel besonders einfach und problemlos verarbeitet werden. Ferner können durch das erfindungsgemäße Verfahren Mikropartikel erhalten werden, die auf bestimmte Bedürfnisse abgestimmt sind. So können Mikropartikel mit den unterschiedlichsten Freisetzungsmechanismen erhalten werden. Hierzu gehören unter anderem Mechanismen, die auf einem enzymatischen Abbau des Trägerpolymers oder einer pHselektiven Öffnung des Trägerpolymers basieren; temperatur- bzw. lösungsmittelgesteuerte Vorgänge, Einwirkung von Energie auf die Mikropartikel, zum Beispiel Bestrahlung der Partikel mit elektromagnetischer Strahlung, Bestrahlung mit Ultraschall und/oder Einwirkung von Scherkräften. Darüber

hinaus können durch das erfindungsgemäße Verfahren Partikel erhalten werden, die einen hohen Anteil an zu verkapselnder Substanz aufweisen. Die Anlagen zur Durchführung des erfindungsgemäßen Verfahrens erfordern hierbei im Allgemeinen nur sehr geringe Investitions- und Betriebskosten, da die Anlagen auch bei Normaldruck betrieben werden können und vielfach keine explosiven Gemische gebildet werden, wobei im Allgemeinen auf den Einsatz von gesundheitlich bedenklichen Stoffen verzichtet werden kann. Beim Betrieb benötigen die Anlagen im Allgemeinen nur geringe Mengen an Energie. Darüber hinaus weisen die Anlagen vielfach eine geringe Komplexität auf, so dass die Wartungskosten niedrig sind und die Anlagen einfach und sicher gesteuert werden können.

Durch das erfindungsgemäße Verfahren sind Mikropartikel erhältlich. Der Begriff Mikropartikel ist in der Fachwelt bekannt. Im Rahmen der vorliegenden Erfindung werden hierunter vorzugsweise Präparate verstanden, in der eine zu verkapselnde Substanz bevorzugt durch eine nicht kovalente Weise mit dem Trägerpolymer verbunden ist. Dies kann beispielsweise durch ionische oder polare Wechselwirkungen oder durch Van-der-Waals Kräfte erfolgen.

Aufgrund der Wechselwirkung von Trägerpolymer und zu verkapselnder Substanz können sich die Mikropartikel von einer konventionellen Mischung dieser Komponenten unterscheiden.

Diese Wechselwirkung kann auf bekannte Weise gemessen werden. Je nach niedermolekularer Substanz sind hierfür vielfach spektroskopische Methoden geeignet. Beispielsweise können teilweise Verschiebungen im Infrarotspektrum beobachtet werden.

Des Weiteren können die erfindungsgemäßen Mikropartikel gegenüber einer konventionellen Mischung eine verzögerte Freisetzung der niedermolekularen Substanz in ein Medium zeigen, welches von der zu verkapselnden Substanz des Präparats verschieden ist. Die verzögerte Freisetzung kann gemäß der von Smirnova, I.; Suttiruengwong, S.; Arlt, W. "Feasibility study of hydrophilic and hydrophobic silica aerogels as drug delivery systems"; Journal of Non-Crystalline Solids (2004) 54-60, beschriebenen Verfahren gemessen werden.

Im Allgemeinen beträgt der Zeitunterschied, um eine identische Konzentration der niedermolekularen Substanz in dem Medium zu erhalten, in das die niedermolekulare Substanz freigesetzt wird, mindestens 1 Minute, bevorzugt mindestens 5 Minuten. Hierbei bezieht sich dieser Zeitunterschied auf die Messung von Mikropartikeln, die gemäß der vorliegenden Erfindung erhältlich sind, und die Messung einer konventionellen Mischung dieser Komponenten unter identischen Bedingungen, wobei diese Messung so ausgeführt wird, dass eine verzögerte Freisetzung vorliegt. Der Begriff "verzögerte Freisetzung" bedeutet in diesem Zusammenhang, dass die Messung nicht unter Bedingungen durchgeführt wird, unter denen die Mikropartikel die zu verkapselnde Substanz möglichst schnell freisetzen. Dementsprechend sollte das Medium kein Lösungsmittel für das Trägerpolymer darstellen, das als Komponente der Mikropartikel dient. Des Weiteren sollten die Messungen bei Temperaturen durchgeführt werden, die unterhalb der Verfestigungstemperatur des Trägerpolymeren liegt. Diese Bedingungen sind dem Fachmann bei Kenntnis dieser Anmeldung geläufig. Die Werte der konventionellen Mischung können auch durch getrennte Zugabe der Komponenten ermittelt werden.

Nach einer besonderen Ausführungsform liegen die Mikropartikel verkapselt vor, wobei der Begriff "Verkapselung" in der Fachwelt bekannt ist. Im verkapselten Präparat kann beispielsweise die zu verkapselnde Verbindung in einer Hülle eingebettet werden, die Trägerpolymer umfasst. Dies kann beispielsweise durch eine Matrixverkapselung und/oder eine Kern-Hülle-Verkapselung erfolgen.

Unter dem Begriff Matrixverkapselung werden Verfahren verstanden, durch die insbesondere Präparate erhältlich sind, die eine oder mehrere verkapselte Verbindungen umfassen, die in einem kontinuierlichen oder diskontinuierlichen Verkapselungsmaterial verteilt vorliegen. Hierbei kann das verkapselte Material so fein verteilt sein, dass eine homogene Mischung aus Hüllmaterial und Kernmaterial vorliegt. Kern-Hülle-Verkapselung bezeichnet Verfahren, durch die Präparate mit einem oder mehreren Kernen und einer oder mehreren Hüllen erhältlich sind. Die Hülle umfasst vorzugsweise Trägerpolymere.

Die Form der Partikel ist an sich unkritisch, wobei die Partikel jedoch vorzugsweise eine sphärische Form aufweisen.

Der Begriff sphärisch bezeichnet im Rahmen der vorliegenden Erfindung, dass die Partikel vorzugsweise eine kugelförmige Gestalt aufweisen, wobei dem Fachmann offensichtlich ist, dass aufgrund der Herstellungsmethoden auch Partikel mit anderer Gestalt enthalten sein können, oder dass die Form der Partikel von der idealen Kugelgestalt abweichen kann.

Dementsprechend bedeutet der Begriff sphärisch, dass das Verhältnis von der größten Ausdehnung der Partikel zur geringsten Ausdehnung maximal 4, vorzugsweise maximal 2 beträgt, wobei diese Ausdehnungen jeweils durch den Schwerpunkt der Partikel gemessen werden. Vorzugsweise sind mindestens 70, besonders bevorzugt mindestens 90 %, bezogen auf die Zahl der Partikel, sphärisch.

Gemäß einem besonderen Aspekt der vorliegenden Erfindung können die Mikropartikel vorzugsweise eine Größe im Bereich von 1 bis 1000 μm, insbesondere bevorzugt 3 bis 800 μm, besonders bevorzugt 7 bis 700 μm und ganz besonders bevorzugt 10 bis 500 μm aufweisen. Diese Größe bezieht sich auf das Zahlenmittel der Partikel, wobei die größte Ausdehnung der Partikel zu wählen ist. Diese Größen können über mikroskopische Aufnahmen ermittelt werden.

Des Weiteren weisen bevorzugte Mikropartikel

eine besonders enge Partikelgrößenverteilung auf. So liegen bevorzugt mindestens 80 Gew.-% der Partikel innerhalb eines Größenbereichs von höchstens 200 μm, vorzugsweise höchstens 100μm, besonders bevorzugt höchstens 50 μm.

Gemäß einem besonderen Aspekt der vorliegenden Erfindung weisen vorzugsweise 90% der Partikel eine Größe im Bereich von 1 bis 1000 μm, insbesondere bevorzugt 3 bis 800 μm, besonders bevorzugt 7 bis 700 μm und ganz besonders bevorzugt 10 bis 500 μm auf.

Je nach Anwendungsgebiet kann dementsprechend eine vorgegebene Partikelgröße bei einer engen Partikelgrößenverteilung hergestellt werden. So können bestimmte Mikropartikel eine Größe im Bereich von 5 μm bis 200 μm, vorzugsweise im Bereich von 10 μm bis 60 μm liegen, wobei dieser Größenbereich insbesondere bei kosmetischen Anwendungen Interesse finden kann. Andere Mikropartikel können hingegen eine Größe im Bereich von 150 μm bis 750 μm, bevorzugt 250 μm bis 500 μm aufweisen, wobei diese Größe insbesondere im Bereich der Aroma- oder Geschmacksstoffe Anwendung finden kann, falls eine Freisetzung über eine mittlere Zeitdauer erwünscht ist. Falls eine längere Freisetzungszeit erwünscht ist, können größere Mikropartikel eingesetzt werden, die vorzugsweise eine Größe im Bereich von 450 μm bis 1000 μm, besonders bevorzugt 800 μm bis 1000 μm aufweisen können. Des Weiteren können die größeren Mikropartikel auch Anwendung finden, falls beispielsweise das Problem von Stäuben vermindert werden soll.

Die zu verkapselnden Substanzen können aus den erfindungsgemäß erhältlichen Mikropartikeln auf eine gewünschte Weise freigesetzt werden. Beispielsweise kann ein enzymatischer Abbau eingesetzt werden, um die niedermolekulare Substanz freizusetzen. Hierbei kann der Freisetzungszeitraum durch die Abbaurate gesteuert werden.

Weiterhin kann die Freisetzung über eine Änderung des pH-Wertes, der Temperatur, und der Art des Mediums gezielt gesteuert werden. Ferner kann eine gezielte Freisetzung durch die Einwirkung von Energie auf die Mikropartikel, zum Beispiel Bestrahlung der Partikel mit elektromagnetischer Strahlung, Bestrahlung mit Ultraschall und/oder Einwirkung von Scherkräften erfolgen. Diese Freisetzungsmechanismen können einzeln oder in Kombination von zwei, drei oder mehr dieser Methoden eingesetzt werden.

Die Art des Mediums kann beispielsweise über die Zugabe von Lösungsmittel geändert werden.

Als Lösungsmittel zur Variation des Mediums können unter anderem Wasser, Alkohole wie Ethanol oder Isopropanol komprimiertes $CO_2$, komprimiertes Propan, Tetrahydrofuran, Toluol, Aceton, Benzoylperoxid, wässerige HCl-Lösungen, Hexan, Essigsäure, Ethandiol, Dichlormethan, Dichlorethan oder ionische Flüssigkeiten eingesetzt werden.

Beispielsweise kann die Wirkstofffreisetzung insbesondere über die Dicke der Trägerpolymerhülle, die die niedermolekulare Substanz bzw. den Wirkstoff umgibt, und/oder den Funktionalisierungsgrad / Hydrophobisierungsgrad bzw. die Hydroxyzahl des Trägerpolymers gesteuert werden. Die Dicke der Polymerhülle kann über eine erfindungsgemäße Variation der Herstellungsparameter gezielt eingestellt werden. So kann ein hoher Anteil an Trägerpolymer in den Mikropartikeln zu einer dickeren Polymerhülle und vielfach auch zu scherstabileren Partikeln führen, die erst bei einer hohen Energieeinwirkung die zu verkapselnden Substanzen freisetzen. Ähnlich führen Partikel mit einem hohen Anteil an Trägerpolymer vielfach zu einer Freisetzung über einen längeren Zeitraum, falls die Freisetzung über eine Variation des Lösungsmittels oder über einen enzymatischen Abbau erfolgt. Ähnlich kann die Morphologie der Partikel einen Einfluss auf das Freisetzungsprofil haben, die unter anderem über die Verteilung der zu verkapselnden Substanz in der Polymerschmelze beeinflusst werden kann.

Der Freisetzungszeitraum ist umso größer, je dicker die Trägerpolymerhülle ist. Die Dicke der Trägerpolymerhülle lässt sich insbesondere durch Erhöhung der Polymerkonzentration in der Ausgangsmischung (vorzugsweise bestehend aus mindestens einem hyperverzweigten Polymer und einer niedermolekularen Substanz) vergrößern.

Gemäß dem erfindungsgemäßen Verfahren wird eine Polymerschmelze hergestellt. Der Begriff Polymerschmelze bezeichnet einen fließfähigen Zustand einer Zusammensetzung, die mindestens ein Trägerpolymer und mindestens eine zu verkapselnde Substanz umfasst. Vorzugsweise liegt die Viskosität der Polymerschmelze im Bereich von 50 mPa*s bis 5000 Pa*s , besonders bevorzugt im Bereich von 100 mPa*s bis 1000 Pa*s, wobei diese Größe mittels Rotationsviskosimetrie bestimmt werden kann. Der fließfähige Zustand ist hierbei unter anderem von der Temperatur abhängig, so dass viele bekannte Trägerpolymere durch Erhitzen in eine Polymerschmelze überführt werden können. Bevorzugt werden die zuvor genannten Viskositätsbereiche der Polymerschmelze bei einer Temperatur im Bereich von 10 bis 200 °C, besonders bevorzugt im Bereich von 50 bis 150 °C gemessen. Gemäß einem besonderen Aspekt der vorliegenden Erfindung kann die Viskosität der Polymerschmelze im Bereich von 100 mPa*s bis 1000 Pa*s, insbesondere 100 mPa*s bis 100 Pa*s, liegen, wobei diese Größe mittels Rotationsviskosimetrie bei 110°C und 30 $s^{-1}$ zwischen zwei 20 mm Platten gemessen werden kann.

Die erfindungemäß herzustellende Polymerschmelze umfasst mindestens ein Trägerpolymer. Die Art des Trägerpolymeren ist von der Anwendung der Mikropartikel abhängig, wobei im Prinzip jedes bekannte Polymer eingesetzt werden kann. Der Begriff Trägerpolymer dient im Rahmen der vorliegenden Erfindung insbesondere zur Abgrenzung der als Schutzpolymer verwendeten Verbindungen gegenüber der zu verkapselnden Substanz, die ebenfalls ein Polymer sein kann. Vorzugsweise bildet das Trägerpolymer die Matrixphase, in der die zu verkapselnde Substanz verteilt wird.

Bevorzugte Trägerpolymere weisen eine Schmelztemperatur oder eine Glasübergangstemperatur von mindestens -30°C, bevorzugt mindestens -10 °C, besonders bevorzugt mindestens 0°C, insbesondere mindestens 25°C, besonders bevorzugt mindestens 35 °C und ganz besonders bevorzugt mindestens 40°C auf. Gemäß einem besonderen Aspekt der vorliegenden Erfindung kann die Schmelztemperatur oder die Glasübergangstemperatur des Trägerpolymers vorzugsweise höchstens 150°C, insbesondere bevorzugt höchstens 100°C, besonders bevorzugt höchstens 80°C und ganz besonders bevorzugt höchstens 60°C betragen. Die Schmelztemperatur oder die Glasübergangstemperatur kann mittels Differential Scanning Calorimetry (DSC) erfolgen, z.B. mit dem Apparat Mettler DSC 27 HP und einer Heizrate von 10°C/min. Hierbei ist festzuhalten, dass amorphe Polymere im Allgemeinen lediglich eine Glasübergangstemperatur aufweisen, wohingegen kristalline Polymere eine Schmelztemperatur zeigen. Teilkristalline Polymere können sowohl eine Glasübergangstemperatur als auch eine Schmelztemperatur zeigen, wobei in diesem Fall die Temperatur ausschlaggebend ist, bei der die Partikel keine Agglomeration zeigen. Falls die Oberfläche im Wesentlichen kristallin ist, so ist der Schmelzpunkt dieser Bestandteile entscheidend.

Vorzugsweise weisen die Trägerpolymere ein Molekulargewicht von mindestens 1000 g/mol, besonders bevorzugt mindestens 5000 g/mol und ganz besonders bevorzugt mindestens 10000 g/mol auf. Vorzugsweise beträgt das Molekulargewicht höchstens 10000000 g/mol, besonders bevorzugt höchstens 700000 g/mol und ganz besonders bevorzugt höchstens 500000 g/mol. Diese Größe bezieht sich auf das Gewichtsmittel des Molekulargewichts (Mw), welches mittels Gelpermationschromatographie gemessen werden kann.

Die Polydispersität Mw/Mn bevorzugter Trägerpolymere liegt vorzugsweise im Bereich von 1,01 bis 10,0, besonders bevorzugt im Bereich von 1,10 bis 5,0 und ganz besonders bevorzugt im Bereich von 1,2 bis 3,0, wobei das Zahlenmittel des Molekulargewichts (Mn) ebenfalls durch GPC erhalten werden kann.

Die Viskosität des Trägerpolymeren liegt vorzugsweise im Bereich von 50 mPa*s bis 1000 Pa*s, besonders bevorzugt im Bereich von 100 mPa*s bis 100 Pa*s, wobei diese Größe mittels Rotationsviskosimetrie bei 110°C und 30 $s^{-1}$ zwischen zwei 20 mm Platten gemessen werden kann. Diese Viskosität kann im Allgemeinen ohne Lösungsmittel bestimmt werden.

Gemäß einem bevorzugten Aspekt der vorliegenden Erfindung kann ein hydrophiles Polymer als Trägerpolymer eingesetzt werden.

Im Rahmen der vorliegenden Erfindung wird unter dem Begriff "hydrophil" die Eigenschaft verstanden, dass das Polymer in der Lage ist, einen hohen Anteil an Wasser aufzunehmen. Gemäß einem bevorzugten Aspekt der vorliegenden Erfindung ist das hydrophile Polymer in Wasser löslich. Vorzugsweise beträgt die Löslichkeit in Wasser bei 90°C mindestens 10 Massenprozent, besonders bevorzugt mindestens 20 Massenprozent. Die Messung kann gemäß der sogenannten Kolbenmethode erfolgen, wobei die Wasserlöslichkeit der reinen Substanz gemessen wird.

Bei dieser Methode wird die Substanz (Feststoffe müssen pulverisiert werden) bei einer Temperatur in Wasser aufgelöst, die leicht über der Prüftemperatur liegt. Wenn die Sättigung erreicht ist, wird die Lösung abgekühlt und bei der Prüftemperatur gehalten. Die Lösung wird gerührt, bis das Gleichgewicht erreicht ist. Alternativ kann die Messung unmittelbar bei der Prüftemperatur durchgeführt werden, wenn durch entsprechende Probenahme gesichert ist, dass das Sättigungsgleichgewicht erreicht ist. Dann wird die Konzentration der Prüfsubstanz in der wässrigen Lösung, die keine ungelösten Substanzteilchen enthalten darf, mit einer geeigneten Analysenmethode bestimmt.

Gemäß einer weiteren bevorzugten Ausführungsform kann ein hydrophobes Polymer als Trägerpolymer eingesetzt werden. Die Wasserlöslichkeit eines hydrophoben Trägerpolymers beträgt vorzugsweise höchstens 10 Massenprozent, besonders bevorzugt höchstens 7 Massenprozent und ganz besonders bevorzugt höchstens 5 Massenprozent, gemessen nach der zuvor dargelegten Kolbenmethode bei 40°C.

Gemäß einem besonderen Aspekt der vorliegenden Erfindung werden bevorzugt Trägerpolymere eingesetzt, die eine Zulassung gemäß Community Register of Feed Additives pursuant to Regulation (EC) No 1831/2003, Rev. 4 Status: Released 29 May 2006 aufweisen.

Nach einer weiteren Ausführungsform der vorliegenden Erfindung können vorzugsweise Trägerpolymere eingesetzt werden, die eine Zulassung gemäß FDA (Food and Drug Administration) aufweisen.

Zu den bevorzugten Trägerpolymeren gehören Polyester, beispielsweise Polyethylenterephthalate oder Polybutylenterephthalate; Polyacrylate, beispielsweise Polymethylmethacrylate; Polyamide; Polyvinylalkohole; Polyethylenglykole; hochverzweigte Polymere, insbesondere Dendrimere sowie hyperverzweigten Polymere und/oder Biopolymere. Diese Polymere können als Homopolymere oder als Copolymere eingesetzt werden, wobei die Copolymere einen hohen Anteil an Wiederholungseinheiten aufweisen, die in den zuvor dargelegten Polymeren enthalten sind. Ein hoher Anteil bedeutet, dass die Copolymere vorzugsweise mindestens 30 Gew.-%, besonders bevorzugt mindestens 50 Gew.-% und besonders bevorzugt mindestens 80 Gew.-% an Wiederholungseinheiten aufweisen, die in den zuvor dargelegten Polymeren enthalten sind. Des Weiteren können diese Polymere auch als Mischungen, so genannte Blends von zwei der mehreren der zuvor dargelegten Homo- oder Copolymere eingesetzt werden.

Polyacrylate sind in der Fachwelt bekannt. Hierunter werden Homopolymere oder Copolymere auf Basis von Acrylaten verstanden. Diese Polymere weisen vorzugsweise mindestens 30 Gew.-%, insbesondere bevorzugt mindestens 50 Gew.-%, besonders bevorzugt mindestens 80 Gew.-% und ganz besonders bevorzugt mindestens 95 Gew.-% an Acrylaten auf. Der Begriff Acrylat umfasst Acrylsäure oder Methacrylsäure sowie Monomere, die von Acrylsäure oder Methacrylsäure abgeleitet sind. Zu diesen gehören insbesondere Ester dieser Säuren, beispielsweise Methylmethacrylat, Ethylmethacrylat, Butylmethacrylat, Hexylmethacrylat, Methylacrylat, Ethylacrylat, Butylacrylat und/oder Hexylacrylat. Hierbei können auch Mischungen von zwei oder mehreren dieser Monomere eingesetzt werden. Bevorzugte Polyacrylate sind unter der Handelsbezeichnung EUDRAGIT® kommerziell von der Firma Röhm® GmbH erhältlich.

Hochverzweigte, globulare Polymere werden in der Fachliteratur auch als "dendritische Polymere" bezeichnet. Diese aus multifunktionellen Monomeren synthetisierten dendritischen Polymere lassen sich in zwei unterschiedliche Kategorien einteilen, die "Dendrimere" sowie die "hyperverzweigten Polymere" im engeren Sinne. Dendrimere besitzen einen sehr regelmäßigen, radialsymmetrischen Generationenaufbau. Sie stellen monodisperse globulare Polymere da, die - im Vergleich zu hyperverzweigten Polymeren - in Vielschrittsynthesen mit einem hohen Syntheseaufwand hergestellt werden. Dabei ist die Struktur durch drei unterschiedliche Areale charakterisiert: - dem polyfunktionellen Kern, der das Symmetriezentrum darstellt,

- verschiedenen wohldefinierten radialsymmetrischen Schichten einer Wiederholungseinheit (Generation) und - den terminalen Gruppen. Die hyperverzweigten Polymere im engeren Sinne sind im Gegensatz zu den Dendrimeren polydispers und hinsichtlich ihrer Verzweigung und Struktur unregelmäßig. Neben den dendritischen und linearen Einheiten treten - im Gegensatz zu Dendrimeren - in hyperverzweigten Polymeren auch lineare Einheiten auf.

Bezüglich der unterschiedlichen Möglichkeiten zur Synthese von Dendrimeren und hyperverzweigten Polymeren im engeren Sinne sei auf

a) Fréchet J.M.J., Tomalia D.A., Dendrimers and Other Dendritic Polymers, John Wiley &Sons, Ltd., West Sussex, UK 2001 sowie
b) Jikei M., Kakimoto M., Hyperbranched polymers: a promising new class of materials, Prog. Polym. Sci., 26 (2001) 1233-1285 und/oder
c) Gao C., Yan D., Hyperbranched Polymers: from synthesis to applications, Prog. Polym. Sci., 29 (2004) 183-275 verwiesen, die hiermit als Referenzen eingeführt werden und als Teil der Offenbarung der vorliegenden Erfindung gelten.

Im Rahmen der vorliegenden Erfindung werden unter dem Begriff "hyperverzweigtes Polymer" hochverzweigte Polymere verstanden, die sowohl die zuvor beschriebenen Dendrimere als auch die zuvor dargelegten hyperverzweigten Polymeren im engeren Sinne umfassen. Vorzugsweise können erfindungsgemäß die hyperverzweigten Polymere im engeren Sinne eingesetzt werden, die polydispers und hinsichtlich ihrer Verzweigung und Struktur unregelmäßig sind.

Die in diesen Druckschriften beschriebenen hyperverzweigten und hochverzweigten Polymere sind auch im Sinne der vorliegenden Erfindung bevorzugte Trägerpolymere. In diesem Zusammenhang ist es bevorzugt, dass die hyperverzweigten Polymere mindestens 3 Wiederholungseinheiten pro Molekül, vorzugsweise mindestens 10 Wiederholungseinheiten pro Molekül, ferner bevorzugt mindestens 100 Wiederholungseinheiten pro Molekül, zudem bevorzugt mindestens 200 Wiederholungseinheiten und darüber hinaus bevorzugt mindestens 400 Wiederholungseinheiten besitzen, die jeweils mindestens drei, bevorzugt mindestens vier Bindungsmöglichkeiten aufweisen, wobei mindestens 3 dieser Wiederholungseinheiten, besonders bevorzugt mindestens 10 und darüber hinaus bevorzugt mindestens 20 jeweils über mindestens drei, bevorzugt über mindestens vier Bindungsmöglichkeiten mit mindestens drei, bevorzugt mindestens vier weiteren Wiederholungseinheiten verknüpft sind.

Verschiedentlich weisen die hyperverzweigten Polymere maximal 10000, vorzugsweise maximal 5000 und besonders bevorzugt maximal 2500 Weiderholungseinheiten auf.

In einer bevorzugten Ausführungsform weist das hochverzweigte Polymer mindestens drei Wiederholungseinheiten auf, welche jeweils mindestens drei mögliche Bindungsmöglichkeiten aufweisen, wobei mindestens drei dieser Wiederholungseinheiten mindestens zwei mögliche Bindungsmöglichkeiten aufweisen.

Dabei wird unter dem Begriff "Wiederholungseinheit" vorzugsweise eine stets wiederkehrende Struktur innerhalb des hyperverzweigten Moleküls verstanden. Unter dem Begriff "Bindungsmöglichkeit" wird vorzugsweise diejenige funktionelle Struktur innerhalb einer Wiederholungseinheit verstanden, mit der eine Verknüpfung zu einer anderen Wiederholungseinheit möglich ist. Bezogen auf die vorstehend dargestellten Beispiele eines Dendrimers bzw. eines hyperverzweigten Polymers ist die Wiederholungseinheit eine Struktur mit jeweils drei Bindungsmöglichkeiten (X,Y,Z):

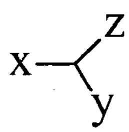

Die Verknüpfung der einzelnen Bindungseinheiten miteinander kann durch Kondensationspolymerisation, durch radikalische Polymerisation, durch anionische Polymerisation, durch kationische Polymerisation, durch Gruppentransferpolymerisation, durch koordinative Polymerisation oder durch Ringöffnungspolymerisation erfolgen.

Die Art des hyperverzweigten Polymers ist im Allgemeinen unkritisch. So können hydrophile und/oder hydrophobe hyperverzweigte Polymere eingesetzt werden. Hierzu gehören insbesondere hyperverzweigte Polyester, hyperverzweigte Polyamide, hyperverzweigte Polyesteramide, hyperverzweigte Polyamidoamine, hyperverzweigte Polypropylenamine und hyperverzweigte Polyetherimide. Derartige Polymere sind an sich bekannt und vielfach beschrieben. Diese Polymere können ionische funktionelle Gruppen enthalten. Derartige Polymere sind unter anderem in EP-A-0630389; WO 97/06825; J. Chem. Soc. Perkin Trans. 1992 (Seiten 2459 - 2469); in Macromolecules 1993, 26, Seiten 4617 - 4623; US 5,041,516; US 5,136,014; US 5,183,862; US 5,196,502; US 5,225,522; US 5,227,462; US 5,362,843; US 5,418,301; WO 98/30604; WO 2004/072153; WO 00/065024; WO 2005/034909; WO 03/037383; WO 00/06267; WO 03/033027; WO0056804A1; WO 93/18079; WO 93/17060; EP 869984; WO 00/59982 und in Macromol. Biosci. 5 (2005) 662-668 beschrieben.

Gemäß einem besonderen Aspekt der vorliegenden Erfindung kann ein hydrophiles hyperverzweigtes Polymer eingesetzt werden.

Das hydrophile hyperverzweigte Polymer weist vorzugsweise eine Hydroxyzahl im Bereich von 400 bis 600 mg KOH/g, besonders bevorzugt im Bereich von 450 bis 550 mg KOH/g auf. Diese Eigenschaft wird gemäß ASTM E222 gemessen. Hierbei wird das Polymer mit einer definierten Menge an Essigsäureanhydrid umgesetzt. Nicht umgesetztes Essigsäureanhydrid wird mit Wasser hydrolysiert. Anschließend wird die Mischung mit NaOH titriert. Die Hydroxyzahl ergibt sich aus dem Unterschied zwischen einer Vergleichsprobe und dem für das Polymer gemessenen Wert. Hierbei ist die Anzahl an Säuregruppen des Polymers zu berücksichtigen.

Vorzugsweise weist ein erfindungsgemäß einzusetzendes hyperverzweigtes Polymer Polyestereinheiten auf. Hyperverzweigte Polymere mit Polyestereinheiten sind insbesondere in EP 0 630 389 dargelegt. Im Allgemeinen weist das hydrophile Polymer eine zentrale Einheit auf die von einem Initiatormolekül mit mindestens 2, vorzugsweise mindestens 3 Hydroxygruppen abgeleitet ist, und Wiederholungseinheiten auf die von Monomeren mit mindestens einer Carbonylgruppe und mindestens 2 Hydroxygruppen abgeleitet sind.

Die Begriffe Initiatormolekül sowie Wiederholungseinheit sind in der Fachwelt weithin bekannt. So können die erfindungsgemäß einzusetzenden hyperverzweigten Polymere beispielsweise durch Polykondensation erhalten werden, wobei ausgehend von einem mehrwertigen Alkohol zunächst die Carbonsäuregruppen der Monomere umgesetzt werden. Hierbei werden Estergruppen gebildet. Da die Monomere mindestens 2 Hydroxygruppen umfassen, weist das Makromolekül nach jeder Umsetzung mehr Hydroxygruppen auf, als vor der Umsetzung.

Vorzugsweise ist das Initiatormolekül ein aliphatisches Polyol, mit vorzugsweise 3, 4, 5, 6, 7 oder 8, besonders bevorzugt 3, 4 oder 5 Hydroxygruppen.

Besonders bevorzugt ist das Initiatormolekül ausgewählt aus Ditrimethylolpropan, Ditrimethylolethan, Dipentaerythrit, Pentaerythrit, alkoxliertem Pentaerythrit, Trimethylolethan, Trimethylolpropan, alkoxyliertem Trimethylolpropan, Glycerin, Neopentylalkohol, Dimethylolpropan und/oder 1,3-Dioxan-5,5-dimethanol.

Gemäß einem besonderen Aspekt der vorliegenden Erfindung sind die Wiederholungseinheiten von Monomeren mit einer Carboxylgruppe und mindestens 2 Hydroxygruppen abgeleitet. Zu diesen bevorzugten Monomeren gehören insbesondere Dimethylpropionsäure, α,α-Bis(hydroxymethyl)buttersäure, α,α,α-Tris (hydroxymethyl)essigsäure, a,a-Bis(hydroxymethyl) valariansäure, α,α-Bis(hydroxy)propionsäure und/oder 3,5-Dihydroxybenzoesäure.

Ganz besonders bevorzugt ist der hydrophile Kern durch Polymerisation von Dimethylolpropionsäure erhältlich, wobei als Initiatormolekül besonders bevorzugt Ditrimethylolpropan, Trimethylolpropan, ethoxyliertes Pentaerthrit, Pentaerthrit oder Glycerin eingesetzt wird.

Gemäß einem weiteren Aspekt der vorliegenden Erfindung können auch hydrophobe hyperverzweigte Polymere eingesetzt werden. Zu den hydrophoben hyperverzweigten Polymeren gehören insbesondere die zuvor dargelegten hyperverzweigten Polymere mit einer geringen Wasserlöslichkeit.

Des Weiteren können hydrophobe hyperverzweigte Polymere beispielsweise aus den zuvor beschriebenen hydrophilen hyperverzweigten Polymeren durch Hydrophobisierung erhalten werden. Hierbei kann angenommen werden, dass diese hyperverzweigten Polymere einen hydrophilen Kern und hydrophobe Endgruppen aufweisen, ohne dass hierdurch eine Beschränkung erfolgen soll.

Gemäß einer bevorzugten Ausführungsform weist der hydrophile Kern vorzugsweise ein Molekulargewicht von mindestens 1500 g/mol, bevorzugt mindestens 2500 g/mol. Diese Größe bezieht sich auf das Gewichtsmittel des Molekulargewichts (Mw), welches mittels Gelpermationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher ein apparenter Meßwert dar.

Vorzugsweise kann der hydrophile Kern eine Glasübergangstemperatur aufweisen, die im Bereich von -40 bis 60 °C, besonders bevorzugt 0 bis 50°C und ganz besonders bevorzugt 10 bis 40°C liegt. Die Glasübergangstemperatur kann durch DSC-Verfahren ermittelt werden, wobei eine Heizrate von 3°C/min verwendet werden kann (DMA Tan δ peak; Netzch DMA 242 3-point bending 1Hz 3°C/min).

Die Hydrophobisierung der Oberfläche kann im Allgemeinen als letzter Reaktionsschritt durch Umsetzung zumindest eines Teils der freien Hydroxygruppen mit vorzugsweise einer langkettigen Carbonsäure erhalten werden.

Vorzugsweise beträgt der Funktionalisierungsgrad des hyperverzweigten Kernmoleküls mit Fettsäure-enthaltenden Bausteinen mindestens 30%, besonders bevorzugt mindestens 40%. Gemäß einem weiteren Aspekt der vorliegenden Erfindung liegt der Funktionalisierungsgrad des hyperverzweigten Kernmoleküls mit Fettsäure-enthaltenden Bausteinen im Bereich von 30 bis 100%, bevorzugt im Bereich von 35 bis 95%, insbesondere bevorzugt im Bereich von 40 bis 90% und ganz besonders bevorzugt im Bereich von 45 bis 85%.

Der Funktionalisierungsgrad bezieht sich auf den Anteil an Hydroxygruppen die bei der Hydrophobisierung umgesetzt werden. Dementsprechend kann der Funktionalisierungsgrad bzw. der Veresterungsgrad mit Fettsäuren über die Messung der Hydroxy-Zahl für das hyperverzweigte Kernmolekül vor der Hydrophobisierungsreaktion und nach der Hydrophobierungsreaktion bestimmt werden.

Neben dem hydrophilen Kern kann das hyperverzweigte Polymer hydrophobe Endgruppen aufweisen. In diesem Zusammenhang bedeutet der Begriff hydrophobe Endgruppen, dass mindestens ein Teil der Kettenenden des hyperverzweigten Polymers hydrophobe Gruppen aufweist. Hierbei kann angenommen werden, dass hierdurch eine zumindest teilweise hydrophobisierte Oberfläche erhalten wird.

Der Begriff hydrophob ist an sich in der Fachwelt bekannt, wobei die Gruppen, die zumindest an einem Teil der Enden der hyperverzweigten Polymere vorhanden sind, für sich betrachtet, eine geringe Wasserlöslichkeit aufweisen.

Gemäß einem besonderen Aspekt wird die Oberfläche durch Gruppen hydrophobisiert, die von Carbonsäuren mit mindestens 6, bevorzugt mindestens 12 Kohlenstoffatomen abgeleitet sind. Die Carbonsäuren weisen vorzugsweise höchstens 40, besonders höchstens 32 Kohlenstoffatome, besonders bevorzugt höchstens 20 Kohlenstoffatome und ganz besonders bevorzugt höchstens 18 Kohlenstoffatome auf. Hierbei können die Gruppen von gesättigten und/oder ungesättigten Fettsäuren abgeleitet sein. Vorzugsweise beträgt der Anteil der Carbonsäuren mit 12 bis 18 Kohlenstoffatomen mindestens 30 Gew.-%, besonders bevorzugt mindestens 50 Gew.-% und ganz besonders bevorzugt mindestens 60 Gew.-%, bezogen auf das Gewicht der zur Hydrophobisierung eingesetzten Carbonsäuren.

Hierzu gehören insbesondere Fettsäuren, die in Leinsamen, Sojabohnen und/oder Tallöl enthalten sind. Besonders geeignet sind Fettsäuren, die einen geringen Anteil an Doppelbindungen aufweisen, beispielsweise Hexadecensäure, insbesondere Palmitoleinsäure, und Octadecensäure, insbesondere Ölsäure.

Bevorzugte Carbonsäuren weisen hierbei einen Schmelzpunkt von mindestens 35 °C, bevorzugt mindestens 40 °C und besonders bevorzugt mindestens 60 °C auf. Dementsprechend werden bevorzugt lineare, gesättigte Carbonsäuren eingesetzt. Hierzu gehören insbesondere Octansäure, Decansäure, Dodecansäure, Tetradecansäure, Hexadecansäure, Heptadecansäure, Octadecansäure, Eicosansäure, Docosansäure und Tetracosansäure. Besonders bevorzugt sind gesättigte Fettsäuren mit 16 bis 22 Kohlenstoffatomen, insbesondere bevorzugt 16 bis 18 Kohlenstoffatomen.

Das hyperverzweigte Trägerpolymer kann vorzugsweise ein Molekulargewicht von mindestens 6000 g/mol, besonders bevorzugt mindestens 7500 g/mol aufweisen. Vorzugsweise beträgt das Molekulargewicht höchstens 30000 g/mol, besonders bevorzugt höchstens 25000 g/mol. Diese Größe bezieht sich auf das Gewichtsmittel des Molekulargewichts (Mw), welches mittels Gelpermationschromatographie gemessen werden kann, wobei die Messung in DMF erfolgt und als Referenz Polyethylenglykole eingesetzt werden (vgl. u.a. Burgath et. al in Macromol.Chem. Phys., 201 (2000) 782-791). Hierbei wird eine Kalibierkurve eingesetzt, die unter Verwendung von Polystyrol-Standards erhalten wurde. Diese Größe stellt daher ein apparenter Meßwert dar.

Die Polydispersität Mw/Mn bevorzugter hyperverzweigter Polymere liegt vorzugsweise im Bereich von 1,01 bis 6,0, besonders bevorzugt im Bereich von 1,10 bis 5,0 und ganz besonders bevorzugt im Bereich von 1,2 bis 3,0, wobei das Zahlenmittel des Molekulargewichts (Mn) ebenfalls durch GPC erhalten werden kann.

Die Viskosität des hyperverzweigten Polymeren liegt vorzugsweise im Bereich von 50 mPas bis 100 Pas, besonders bevorzugt im Bereich von 70 mPas bis 5,00 Pas, wobei diese Größe mittels Rotationsviskosimetrie bei 110°C und 30 $s^{-1}$ zwischen zwei 20 mm Platten gemessen werden kann.

Die Säurezahl des hyperverzweigten Polymers liegt vorzugsweise im Bereich von 0 bis 20 mg KOH/g, besonders bevorzugt im Bereich von 1 bis 15 mg KOH/g und ganz besonders bevorzugt im Bereich von 6 bis 10 mg KOH/g. Diese Eigenschaft kann durch Titration mit NaOH gemessen werden (vgl. DIN 53402).

Des weiteren kann das hyperverzweigte Polymer nach der Hydrophobisierung bevorzugt eine Hydroxyzahl im Bereich von 0 bis 200 mg KOH/g, bevorzugt im Bereich von 1 bis 150 mg KOH/g und ganz besonders bevorzugt im Bereich von 10 bis 140 mg KOH/g aufweisen. Diese Eigenschaft wird gemäß ASTM E222 gemessen. Hierbei wird das Polymer mit einer definierten Menge an Essigsäureanhydrid umgesetzt. Nicht umgesetztes Essigsäureanhydrid wird mit Wasser hydrolysiert. Anschließend wird die Mischung mit NaOH titriert. Die Hydroxyzahl ergibt sich aus dem Unterschied zwischen einer Vergleichsprobe und dem für das Polymer gemessenen Wert. Hierbei ist die Anzahl an Säuregruppen des Polymers zu berücksichtigen. Dies kann durch die Säurezahl erfolgen, die über das zuvor beschriebenen Verfahren bestimmt werden kann.

Der Verzweigungsgrad des hyperverzweigten Polymers kann bevorzugt im Bereich von 20 bis 70%, vorzugsweise 25 bis 60% liegen. Der Verzweigungsgrad ist abhängig von den zur Herstellung des Polymers, insbesondere des hydrophilen Kerns eingesetzten Komponenten sowie der Reaktionsbedingungen. Der Verzweigungsgrad kann gemäß Frey et al. bestimmt werden, wobei dieses Verfahren in D.Hölter, A.Burgath, H.Frey, Acta Polymer, 1997, 48, 30 und H. Magnusson,E. Malmström, A. Hult, M. Joansson, Polymer 2002, 43, 301 dargelegt ist.

Das hyperverzweigte Polymer weist vorzugsweise eine Schmelztemperatur von mindestens -30°C, bevorzugt mindestens -10 °C, besonders bevorzugt mindestens 0°C, insbesondere mindestens 25°C, besonders bevorzugt mindestens 35 °C und ganz besonders bevorzugt mindestens 40°C auf. Gemäß einem besonderen Aspekt der vorliegenden Erfindung kann der Schmelzpunkt des hyperverzweigten Polymers vorzugsweise höchstens 65°C, insbesondere bevorzugt höchstens 60°C, besonders bevorzugt höchstens 57°C und ganz besonders bevorzugt höchstens 55°C betragen. Die Schmelztemperatur kann mittels Differential Scanning Calorimetry (DSC) erfolgen, z.B. mit dem Apparat Mettler DSC 27 HP und einer Heizrate von 10°C/min.

Vorzugsweise besteht das hyperverzweigte Polymer im Wesentlichen aus Wasserstoff, Sauerstoff und Kohlenstoff. Der Begriff im Wesentlichen bedeutet, dass weitere Elemente bis zu höchstens 10 Gew. %, besonders bevorzugt höchstens 5 Gew. % in dem hyperverzweigten Polymer enthalten sind.

Gemäß einem besonderen Aspekt der vorliegenden Erfindung kann das Trägerpolymer enzymatisch abgebaut werden. Diese Eigenschaft kann beispielsweise durch die Verwendung von Trägerpolymeren mit einer großen Anzahl an Estergruppen erzielt werden.

Die Herstellung bevorzugter Polymere ist insbesondere in EP 630 389 dargelegt. Im Allgemeinen kann ein Initiatormolekül mit mindestens einer Verbindung umgesetzt werden, die mindestens 2 Hydroxygruppen sowie mindestens eine Carbonsäuregruppe umfasst. Hierdurch wird ein hydrophiler Kern erhalten, der mit mindestens einer hydrophoben Verbindung, beispielsweise einer langkettigen Carbonsäure umgesetzt werden kann.

Im Allgemeinen wird die Reaktion bei einer Temperatur im Bereich von 0°C bis 300°C, vorzugsweise 100°C bis 250°C durchgeführt, wobei die Umsetzung durch bekannte Veresterungskatalysatoren beschleunigt werden kann. Zu diesen Katalysatoren gehören beispielsweise Lewis- und Brønstedtsäuren, insbesondere p-Toluolsulfonsäure, Methansulfonsäure, Trifluoressigsäure, $BF_3$, $AlCl_3$ und $SnCl_4$; Titanverbindungen, insbesondere Tetrabutyltitanat; Zink- und/oder Zinnpulver.

Vorzugsweise wird bei der Veresterung freigesetztes Wasser aus der Reaktionsmischung entfernt.

Des Weiteren können als Trägerpolymere auch Biopolymere, insbesondere Polysaccharide, wie Cellulose und Stärke, Proteine und Polypeptide sowie Polyester, insbesondere Polyhydroxyalkanoate eingesetzt werden. Diese Trägerpolymere sind an sich bekannt und beispielsweise in Römpp Chemie Lexikon 2. Auflage auf CD-ROM und in Ullmann's Encyclopedia of Industrial Chemistry, 5. Aufl. auf CD-ROM sowie von K. Mohanty, M. Misra, G. Hinrichsen in Macromolecular Materials and Engineering Volume 276-277, Issue 1, Pages 1 - 24, 2000 beschrieben. Zu den Biopolymeren gehören insbesondere Polymere, die in der Natur vorkommen bzw. vorkommen könnten. Des Weiteren werden im Rahmen der vorliegenden Erfindung auch Polymere verstanden, die biologisch abbaubar sind oder aus den natürlichen vorkommenden Polymeren durch Derivatisierung oder sonstige Veränderung, beispielsweise einen gezielten Abbau des Molekulargewichts gewonnen werden können.

Zu den bevorzugten Biopolymeren gehören unter anderem Polyether, insbesondere Polysaccharide, wie Cellulose und Stärke, insbesondere Amylose und Amylopektin, Polyamide, insbesondere Proteine und Polypeptide sowie natürliche vorkommende oder biologisch abbaubare Polyester, insbesondere Polyhydroxyalkanoate, Polylactide, Polygylcolide und Polylactidcoglycolide. Des Weiteren gehören hierzu auch modifizierte Biopolymere, wie alkoxylierte Stärke, beispielsweise Hydroxyethylstärke (HES) und Celluloseacetat.

Neben dem Trägerpolymer umfasst die Polymerschmelze mindestens eine zu verkapselnde Substanz. Die zu verkapselnde Substanz kann eine niedermolekulare oder eine polymere Verbindung sein, die von dem Trägerpolymeren verschieden ist. Bevorzugt wird eine niedermolekulare Substanz eingesetzt.

Die zu verkapselnde Substanz weist vorzugsweise eine Molmasse im Bereich von 15 g/mol bis 1000 g/mol, besonders bevorzugt 30 g/mol bis 800 g/mol und ganz besonders bevorzugt 60 g/mol bis 500 g/mol auf.

Die erfindungsgemäß einzusetzende zu verkapselnde Substanz kann auf einem weiten Gebiet gewählt werden. Hierzu gehören insbesondere Verbindungen, die eine Peroxidgruppe umfassen, Aminosäuren, Katalysatoren, Farbstoffe und/oder Pigmente, Vitamine, Monomere, Geschmacks- und/oder Aromastoffe, biologisch aktive Komponenten, insbesondere ein Arzneimittel, Initiatoren, Persulfate, Silikone, Tenside, Kieselsäuren, Silane, Enzyme und Coenzyme, Lösungsmittel, Füllstoffe, Reaktivvernetzer, Detergentien, Haarfarben, Betonzusatzmittel oder Pflanzenextrakte.

Zu den bevorzugten biologisch aktiven Komponenten gehören insbesondere Peptide, Vitamine und Vitaminvorstufen, Fette und Fettsäuren, Aminosäuren und Aminosäurevorstufen, beispielsweise Kreatin, Zucker und Zuckerderivate, Nukleotide, Nukleinsäuren sowie Vorstufen und Derivate derselben, beispielsweise DNA-

und RNA-Oligomere und -Polymere.

Zu den Vitaminen gehören insbesondere Vitamin A, Vitamine des B-Komplexes, beispielsweise Vitamin B1, Vitamin B2, Vitamin B3 (Folsäure) und Vitamin B12, Vitamin C (Ascorbinsäure), Vitamine des D-Komplexes, insbesondere 7-Dehydrocholesterol, Lumisterol, Calciferol, Ergocalciferol, Cholecalciferol, 22,23-Dihydroergocalciferol und Sitocalciferol, und Vitamin E (Tocopherol) und Vitamin K (Phyllochinon, Menachinon).

Zu den bevorzugten Aminosäuren gehören insbesondere DL-Methionin, L-Lysin, L-Threonin, L-Tryptophan und L-Leucin.

Gegenstand der vorliegenden Erfindung sind dementsprechend auch Präparate, die mindestens ein Trägerpolymer und mindestens eine Aminosäure umfassen. Diese Präparate zeigen die Eigenschaften und Vorteile der zuvor dargelegten Mikropartikel. So zeichnen sich diese Präparate insbesondere durch eine hohe Scherstabilität, eine lange Lagerfähigkeit und ein steuerbares Freisetzungsverhalten aus.

Vorzugsweise ist das mindestens eine Aminosäure umfassende Präparat partikelförmig, besonders bevorzugt sphärisch, wie bereits im Zusammenhang mit den Mikropartikeln dargelegt, wobei hierauf Bezug genommen wird. Gemäß einem besonderen Aspekt können die Partikel eine Größe im Bereich von 0,1 $\mu$m bis 2000$\mu$m, besonders bevorzugt 1 $\mu$m bis 1000 $\mu$m, insbesondere bevorzugt 3 bis 800 $\mu$m, besonders bevorzugt 7 bis 700 $\mu$m und ganz besonders bevorzugt 10 bis 500 $\mu$m aufweisen.

Des Weiteren weisen bevorzugte Präparate, die mindestens eine Aminosäure umfassen, eine besonders enge Partikelgrößenverteilung auf. So liegen bevorzugt mindestens 80 Gew.-% der Partikel innerhalb eines Größenbereichs von höchstens 200 $\mu$m, besonders bevorzugt höchstens 100 $\mu$m und ganz besonders bevorzugt höchstens 50 $\mu$m.

Das mindestens eine Aminosäure umfassende Präparat kann einen überraschend hohen Anteil an Aminosäuren aufweisen. Gemäß einem besonderen Aspekt der vorliegenden Erfindung kann das Gewichtsverhältnis von Trägerpolymer zu Aminosäure vorzugsweise im Bereich von 40:1 bis 0,5:1, besonders bevorzugt im Bereich von 20:1 bis 2:1 liegen. Der Beladungsgrad kann vorzugsweise in einem Bereich von 1% bis 99%, besonders bevorzugt 5% bis 90% und ganz besonders 10 bis 30% liegen, wobei der Beladungsgrad durch den Gewichtsanteil der Aminosäure am Gesamtgewicht des Präparats gegeben ist.

Das mindestens eine Aminosäure umfassende Präparat kann sämtliche der zuvor dargelegten Trägerpolymere umfassen. Vorzugsweise wird als Trägerpolymer mindestens ein Polyacrylat eingesetzt. Gemäß einem besonderen Aspekt der vorliegenden Erfindung wird als Trägerpolymer zur Herstellung der Aminosäuren enthaltenden Präparate vorzugsweise kein hyperverzweigtes Polymere mit einem hydrophilen Kern und hydrophoben Endgruppen, besonders bevorzugt kein hyperverzweigtes Polymer eingesetzt.

Zu den bevorzugten Verbindungen mit einer Peroxidgruppe gehören insbesondere $H_2O_2$, Persulfate und/oder organische Peroxide.

Zu den Monomeren gehören insbesondere Olefine, beispielsweise 1-Buten, 1-Hexen, Norbornen;

Vinylhalogenide, wie beispielsweise Vinylchlorid, Vinylfluorid, Vinylidenchlorid und Vinylidenfluorid;

Vinylester, wie Vinylacetat;

Styrol, substituierte Styrole mit einem Alkylsubstituenten in der Seitenkette, wie z. B. $\alpha$-Methylstyrol und $\alpha$-Ethylstyrol, substituierte Styrole mit einem Alkylsubstitutenten am Ring, wie Vinyltuluol und p-Methylstyrol, halogenierte Styrole, wie beispielsweise Monochlorstyrole, Dichlorstyrole, Tribromstyrole und Tetrabromstyrole;

Heterocyclische Vinylverbindungen, wie 2-Vinylpyridin, 3-Vinylpyridin, 2-Methyl-5-vinylpyridin, 3-Ethyl-4-vinylpyridin, 2,3-Dimethyl-5-vinylpyridin, Vinylpyrimidin, Vinylpiperidin, 9-Vinylcarbazol, 3-Vinylcarbazol, 4-Vinylcarbazol, 1-Vinylimidazol, 2-Methyl-1-vinylimidazol, N-Vinylpyrrolidon, 2-Vinylpyrrolidon, N-Vinylpyrrolidin, 3-Vinylpyrrolidin, N-Vinylcaprolactam, N-Vinylbutyrolactam, Vinyloxolan, Vinylfuran, Vinylthiophen, Vinylthiolan, Vinylthiazole und hydrierte Vinylthiazole, Vinyloxazole und hydrierte Vinyloxazole;

Vinyl- und Isoprenylether;

Maleinsäure und Maleinsäurederivate, wie beispielsweise Mono- und Diester der Maleinsäure, Maleinsäureanhydrid, Methylmaleinsäureanhydrid, Maleinimid, Methylmaleinimid;

Fumarsäure und Fumarsäurederivate, wie beispielsweise Mono- und Diester der Fumarsäure;

Diene wie beispielsweise Divinylbenzol;

sowie Acrylate und Methacrylate, die nachfolgend als (Meth)acrylate bezeichnet werden.

Diese umfassen beispielsweise

(Meth)acrylate, die sich von gesättigten Alkoholen ableiten, wie Methyl(meth)acrylat, Ethyl(meth)acrylat, n-Propyl(meth)acrylat, iso-Propyl(meth)acrylat, n-Butyl(meth)acrylat, tert-Butyl(meth)acrylat, Pentyl (meth)acrylat, Hexyl(meth)acrylat, 2-Ethylhexyl(meth)acrylat, Heptyl(meth)acrylat, 2-tert.-Butylheptyl (meth)acrylat, Octyl(meth)acrylat, 3-iso-Propylheptyl(meth)acrylat, Nonyl(meth)acrylat, Decyl(meth) acrylat, Undecyl(meth)acrylat, 5-Methylundecyl (meth)acrylat, Dodecyl(meth)acrylat, 2-Methyldodecyl(meth)acrylat, Tridecyl(meth)acrylat, 5-Methyltridecyl(meth)acrylat, Tetradecyl(meth)acrylat, Pentadecyl(meth)acrylat, Hexadecyl(meth)acrylat, 2-Methylhexadecyl(meth)acrylat, Heptadecyl(meth)acrylat, 5-iso-Propylheptadecyl(meth)acrylat, 4-tert.-Butyloctadecyl(meth)acrylat, 5-Ethyloctadecyl(meth) acrylat, 3-iso-Propyloctadecyl(meth)acrylat, Octadecyl(meth)acrylat, Nonadecyl(meth)acrylat, Eicosyl(meth)acrylat, Cetyleicosyl(meth)acrylat, Stearyleicosyl(meth)acrylat, Docosyl(meth)acrylat und/ oder Eicosyltetratriacontyl-(meth)acrylat;

Cycloalkyl(meth)acrylate, wie Cyclopentyl(meth) acrylat, 3-Vinylcyclohexyl(meth)acrylat, Cyclohexyl (meth)acrylat, Bornyl(meth)acrylat;

(Meth)acrylate, die sich von ungesättigten Alkoholen ableiten, wie 2-Propinyl(meth)acrylat, Allyl(meth) acrylat, Vinyl(meth)acrylat und/oder Oleyl(meth) acrylat;

Aryl(meth)acrylate, wie Benzylmethacrylat oder Phenylmethacrylat, wobei die Arylreste jeweils unsubstituiert oder bis zu vierfach substituiert sein können;

Methacrylate von halogenierten Alkoholen, wie 2,3-Dibromopropylmethacrylat, 4-Bromophenylmethacrylat, 1,3-Dichloro-2-propylmethacrylat, 2-Bromoethylmethacrylat, 2-Iodoethylmethacrylat, Chloromethylmethacrylat.

Des Weiteren können bevorzugte zu verkapselnde Substanzen aus dem Bereich der Detergenzien sowie Additive, die in Haarwaschmitteln sowie Haarfärbemitteln eingesetzt werden, beispielsweise Acetaminophen, acetylierter Lanolinalkohol, Achillea Millefolium, Aesculus Hippocastanum, Agave Rigida, Aloe Barbadensis, Lumina, Aluminumchlorhydrat, Aluminumformiat, Aluminumhydroxid, Magnesiumhydroxid, Stearate, insbesondere Aluminiumstearat und Magnesiumstearat, Aluminumsilikat, Aluminumtristearat, Aminomethylpropanol, Ammoniak, Ammoniumbicarbonat, Ammoniumsulfat, Ammoniumthioglykolsäure, Ammoniumthiolactate, Amodimethicone, Anthemis Nobilis, Arachis Hypogaea.

Zu den bevorzugten Katalysatoren gehören insbesondere Metall umfassende Katalysatoren die zur Hydrierung bzw. Hydrogenierung eingesetzt werden, beispielsweise zur Härtung von Fettsäuren, Nitrilen und Polyolhydrogenierung, Hydrogenierung von Harzen, Aromaten und Weißölen; selektive Hydrogenierung von Acetylenen und Dienen sowie selektive Hydrogenierung von AMS zu Cumol eingesetzt werden.

Des Weiteren gehören hierzu Katalysatoren, die zur Oxidation eingesetzt werden, beispielsweise zur selektiven Oxidation zur Herstellung von Ethylenoxid und Vinylacetatmonomeren; Reinigung von Abgasen aus Prozessen beispielsweise zum Entfernen von CO und VOCs; Reinigung von Abgasen, die halogenierte Kohlenwasserstoffe umfassen; sowie die Katalysatoren zur Reinigung von technischen Gasen.

Diese Katalysatoren umfassen im Allgemeinen Metalle, beispielsweise Nickel, Kobalt, Kupfer, Molybdän, Chrom, Eisen, sowie Platinmetalle, beispielsweise Rhodium, Palladium, Platin. Derartige Katalysatoren sind an sich bekannt und können vielfach kommerziell erhalten werden. Insbesondere gehören hierzu Produkte unter der Handelsbezeichnung catASium® sowie cataXium® von Degussa.

Beispiele für diese Katalysatoren sind unter anderem

(-)-2,3-Bis[(-2R,5R)-2,5-Dimethylphospholanyl] maleinsäureanhydrid (1,5-cyclooctadien)rhodium(I) tetrafluoroborat (catASium® M(R)Rh);

(-)-2,3-bis[(2R,5R)-2,5-dimethylphospholanyl]maleinsäure-N-methylimid (1,5-cyclooctadien rhodium (I) tetrafluoroborat (catASium® MN(R)Rh);

(+)-2,3-bis[(2S,5S)-2,5-dimethylphospholanyl] maleinsäureanhydrid (1,5-cyclooctadien) rhodium(I) tetrafluoroborat (catASium® M(S)Rh);

(+)-2,3-Bis[(2S,5S)-2,5-dimethylphospholanyl] maleinsäure-N-methylimid (1,5-cyclooctadien) rhodium (I) tetrafluoroborat (catASium® MN(S)Rh);

(+)-(3R,4R)-Bis(diphenylphosphino)-1-benzylpyrrolidin (catASium® D(R));

(+)-(3R,4R)-Bis(diphenylphosphino)-1-benzylpyrrolidin (1,5-cyclooctadien) rhodium(I) tetrafluoroborat (catASium® D(R)Rh);

Butyldi-1-adamantylphosphin (cataCXium® A);

Benzyldi-1-adamantylphosphin (cataCXium® ABn);

trans-Di(mu-acetato)bis[o-(di-o-tolylphosphino) benzyl]dipalladium (II) (cataCXium® C);

N-Phenyl-2-(dicyclohexylphosphino)pyrrol (cataCXium® PCy); N-Phenyl-2-(di-t-butylphosphino)pyrrol (cataCXium® PtB); 1-(Methoxyphenyl)-(dicyclohexylphosphino)pyrrol (cataCXium® POMeCy) ; 1-(2,4,6-Trimethylphenyl)-2-(dicyclohexylphosphino)imidazol (cataCXium® PICy).

In Bezug auf die Katalysatoren ermöglichen die erfindungsgemäß erhältlichen Mikropartikel eine besonders lange und stabile Haltbarkeit dieser, sowie eine besonders einfache Handhabung. Des Weiteren können die Katalysatoren über einen besonders langen Zeitraum in die Reaktionsmischung freigesetzt werden.

Des Weiteren können die erfindungsgemäßen Präparate als zu verkapselnde Substanz insbesondere Geschmacksstoffe, Aromastoffe, natürliche Extrakte, geschmacksverstärkende Verbindungen, naturidentische Aromastoffe sowie Enzym modifizierte Nahrungsmittelzusätze umfassen.

Zu den Aromastoffen gehören insbesondere Ketone, Aldehyde, schwefelhaltige Verbindungen, Carbonsäureester, Alkohole und/oder natürliche Extrakte.

Zu den bevorzugt einzusetzenden Ketonen gehören, beispielsweise Aceton, Acetophenon, 2,6-Dimethyl-4-Heptanon, 3-Decen-2-on, Methylamylketon, Methylethylketon, Methylheptylketon, Methylnonylketon, 4-Methyl-2-pentanon, Methylpropylketon, 1-Methyl-4-isopropenyl-6-cyclohexen-2-on (D,L-Carvon) und/oder Propiophenon.

Zu den bevorzugt einzusetzenden Aldehyden gehören insbesondere Acetaldehyde, Butylaldehyde, Zimtsäurealdehyd, Decanal, Dodecanal, Heptanal, Hexanal, Isobutyraldehyd, E-2-Decenal, E-2-Dodecanal, E-2-Hexanal, E-2-Nonenal, E-2-Octenal, 2,4-Decadienal, 2,4-Dodecadienal, 2,4-Heptadienal, 2,4-Nonadienal und/oder 2,4-Octadienal.

Zu den bevorzugt einzusetzenden schwefelhaltigen Verbindungen gehören unter anderem

Sulfide, wie beispielsweise Dimethyldisulfid, Dimethyltrisulfid, Diphenyldisulfid, Dipropyldisulfid, Dipropyltrisulfid, Ethylmethylsulfid, Isopropyldisulfid, Methylpropyldisulfid, Methylpropyltrisulfid, Methyl-2-thiofuroat, 4-Methylthio-2-butanon, 3-Methylthio-1-hexylacetat, 4-(Methylthio)4-methyl-2-pentanon;

Thiocarbonsäuren und Thiocarbonsäurederivate, insbesondere Thioester, wie beispielsweise Ethyl-3-(methylthio)butyrat, Ethylthioacetat, Methyl-3-(methylthio)propionat, Methylthiobenzoat, Methylthiobutyrat, Methylthiohexanoat, Methylthio-isovalerat, n-Propyl-thioacetat;

Mercaptane, insbesondere Hexylmercaptan, Isoamylmercaptan, Isobutylmercaptan und/oder

Thioketone, beispielsweise Thiomenthon.

Zu den bevorzugt einzusetzenden Carbonsäureester gehören unter anderem Amylacetat, Isoamylacetat, Ethylacetat, Ethyl p-anistat, Ethylformiat, Ethylhexanoat, Ethyloctanoat, Buttersäureester, n-Hexylacetat, n-Hexylcrotonat, Hexylisovalerat, Isoamylbutyrat, Isoamylhexanoat, Isobutylbutyrat, Isobutylpropionat, Methylbenzoat, 2-Methylbutylacetat, Zimmtsäuremethylester, Methyldecanoat, Methylisovalerat, Methyloctanoat, Methylpropionat, Nerylacetat, Nerylisobutyrat, n-Octylacetat, Phenethylacetat, Phenethylisobutyrat, Phenethylisovalerat, Phenethylpropionat, Phenyl-propyl-acetat, Phenyl-propylhexanoat, n-Propylacetat, n-Propylformiat und/oder n-Propylisobutyrat.

Zu den bevorzugt einzusetzenden Alkoholen gehören insbesondere Anisylalkohol, Benzylalkohol, 1-Butanol, 1-Hexanol, Isoamylalkohol, Isobutylalkohol, Nerol, Ethanol, Phenethylalkohol, Propanol, 2-Heptanol, 2-Octanol, 3-Octanol, 2-Nonanol und/oder 3-Hexanol.

Zu den natürlichen Extrakten gehören insbesondere Bananenextrakte, Erdbeerextrakte, Kakaoextrakte, Vanilleextrakte, Kaffeeextrakte, Teeextrakte, Nussextrakte, Rumextrakte, Extrakte von Zitrusfrüchten, Kernextrakte, Apfelextrakte und Gewürzextrakte. Diese Extrakte können vielfach kommerziell erhalten werden. Hierzu gehören insbesondere Cocoa Absolute 14620, Cocoa LC 10167, Cocoa P 11197, Cocoa U88; alle erhältlich von Degussa AG. Natürliche Extrakte sind hierbei Extrakte die aus natürlichen Quellen erhalten werden können oder Eigenschaften aufweisen, die diesen Extrakten ähnlich sind.

Gemäß einem weiteren Aspekt der vorliegenden Erfindung können Naturstoffextrakte als zu verkapselnde Substanz eingesetzt werden. Naturstoffextrakte sind Extrakte, die aus den Naturstoffen gewonnen werden. Zu den bevorzugten Naturstoffextrakten gehören Zusammensetzungen, die durch Extraktion von, Ananas, Apfel, Banane, Bier, Birne, Erdbeere, Zitrusfrüchten, Himbeere, Johannisbeere, Kaffee, Kaffeeöl, Kirsche, Mango, Orangenöl, Passionsfrucht, Rum, Sauerkirsche, Schlehe und/oder Whisky Pure Malt gewonnen werden.

Gegenstand der vorliegenden Erfindung sind dementsprechend auch Präparate, die mindestens ein Trägerpolymer und mindestens einen Naturstoffextrakt umfassen. Diese Präparate zeigen die Eigenschaften und Vorteile der zuvor dargelegten Mikropartikel. So zeichnen sich diese Präparate insbesondere durch eine hohe Scherstabilität, eine lange Lagerfähigkeit und ein steuerbares Freisetzungsverhalten aus.

Vorzugsweise ist das mindestens einen Naturstoffextrakt umfassende Präparat partikelförmig, besonders bevorzugt sphärisch, wie bereits im Zusammenhang mit den Mikropartikeln dargelegt, wobei hierauf Bezug genommen wird. Gemäß einem besonderen Aspekt können die Partikel eine Größe im Bereich von 0,1 $\mu$m bis 2000$\mu$m, besonders bevorzugt 1 $\mu$m bis 1000 $\mu$m, insbesondere bevorzugt 3 bis 800 $\mu$m, besonders bevorzugt 7 bis 700 $\mu$m und ganz besonders bevorzugt 10 bis 500 $\mu$m aufweisen.

Des Weiteren weisen bevorzugte Präparate, die mindestens einen Naturstoffextrakt umfassen, eine besonders enge Partikelgrößenverteilung auf. So liegen bevorzugt mindestens 80 Gew.-% der Partikel innerhalb eines Größenbereichs von höchstens 200 $\mu$m, besonders bevorzugt höchstens 100 $\mu$m und ganz besonders bevorzugt höchstens 50 $\mu$m.

Das mindestens einen Naturstoffextrakt umfassende Präparat kann sämtliche der zuvor dargelegten Trägerpolymere umfassen. Vorzugsweise wird als Trägerpolymer mindestens ein Polyacrylat eingesetzt. Gemäß einem besonderen Aspekt der vorliegenden Erfindung wird als Trägerpolymer zur Herstellung der Naturstoffextrakte enthaltenden Präparate vorzugsweise kein hyperverzweigtes Polymer mit einem hydrophilen Kern und hydrophoben Endgruppen, besonders bevorzugt kein hyperverzweigtes Polymer eingesetzt.

Darüber hinaus können die erfindungsgemäß erhältlichen Mikropartikel natürliche und synthetische Zusatzstoffe wie Nahrungsmitteladditive umfassen, insbesondere 2-Acetyl-3,5(6)-dimethyl-pyrazin, 2-Acetylpyrazin, 2-Acetylthiazol, 2,3-Diethyl-5-methyl-pyrazine, 2,3-Diethyl-pyrazin, 2,3-Dimethyl-pyrazin, 2,5-Dimethylpyrazin, 2,6-Dimethyl-pyrazin, 2-Ethyl-3,5-dimethyl-pyrazin, 2-Ethylfuran, 2-Ethyl-3-methyl-pyrazin, 2-Ethyl-5 (6)-methyl-pyrazin, 3-Ethylpyridin, 2-Methoxy-3-isobutyl-pyrazin, 2-Methoxy-3(5),(6)-methyl-pyrazin, 2-Methoxy-pyrazin, 2-Methylpyrazin, 2-Pentylfuran, 2,3,5-Trimethyl-pyrazin und/oder Compound 1036 (ein allgemein bekanntes Cognacöl Substitut).

Des Weiteren können die erfindungsgemäß erhältlichen Mikropartikel Enzym modifizierte diätische Zusatzstoffe (Enzyme Modified Diary Ingredients; EMDI) umfassen. Diese Zusatzstoffe sind für eine Vielzahl von Nahrungsmitteln erhältlich, beispielsweise als Käsegeschmacksstoffe unter der Handelsbezeichnung CPF® Cheese Paste Flavors, NCF® Powderd Cheesed Flavors, BCF® Liquid Blue Cheese Flavors, FDF® Liquid Cheese Flavors. Des weiteren sind EMDI als Buttergeschmacksstoffe erhältlich, beispielsweise LBO® Butterfat/ Cremepaste Flavors; NBF® Powdered Butterfat Flavors; FDF®/DCF® Liquid Butter Flavors.

Gemäß einer besonderen Ausführungsform der vorliegenden Erfindung können Percarbonate als zu verkapselnde Substanz eingesetzt werden. Zu den Percarbonaten gehören insbesondere Alkalimetallpercarbonate. Alkalimetallpercarbonate, insbesondere Natriumpercarbonat sind vielfach Komplexe von Alkalimetallcarbonat und Wasserstoffperoxid. Beispielsweise wird unter Natriumpercarbonat eine Komplexverbindung mit der Summenformel $2Na2CO_3*3H_2O_2$ verstanden, wobei diese kommerziell erhältlich ist.

Gegenstand der vorliegenden Erfindung sind dementsprechend auch Präparate, die mindestens ein Percarbonat und mindestens ein Trägerpolymer umfassen. Diese Präparate zeigen die Eigenschaften und Vorteile der zuvor dargelegten Mikropartikel. So zeichnen sich diese Präparate insbesondere durch eine hohe Scherstabilität, eine lange Lagerfähigkeit und ein steuerbares Freisetzungsverhalten aus.

Vorzugsweise ist das Präparat partikelförmig, besonders bevorzugt sphärisch, wie bereits im Zusammenhang mit den Mikropartikeln dargelegt, wobei hierauf Bezug genommen wird. Gemäß einem besonderen Aspekt können die Partikel eine Größe im Bereich von 0,1 μm bis 2000μm, besonders bevorzugt 1 μm bis 1000 μm, insbesondere bevorzugt 3 bis 800 μm, besonders bevorzugt 7 bis 700 μm und ganz besonders bevorzugt 10 bis 500 μm aufweisen.

Des Weiteren weisen bevorzugte Präparate eine besonders enge Partikelgrößenverteilung auf. So liegen bevorzugt mindestens 80 Gew.-% der Partikel innerhalb eines Größenbereichs von höchstens 200 μm, besonders bevorzugt höchstens 100 μm und ganz besonders bevorzugt höchstens 50 μm.

Das erfindungsgemäße Präparat kann einen überraschend hohen Anteil an Percarbonat aufweisen. Gemäß einem besonderen Aspekt der vorliegenden Erfindung kann das Gewichtsverhältnis von Trägerpolymer zu Percarbonat vorzugsweise im Bereich von 40:1 bis 0,5:1, besonders bevorzugt im Bereich von 20:1 bis 2:1 liegen. Der Beladungsgrad kann vorzugsweise in einem Bereich von 1% bis 99%, besonders bevorzugt 5% bis 90% und ganz besonders 10 bis 30% liegen, wobei der Beladungsgrad durch den Gewichtsanteil des Percarbonats am Gesamtgewicht des Präparats gegeben ist.

Das erfindungsgemäße Präparat kann sämtliche der zuvor dargelegten Trägerpolymere umfassen. Vorzugsweise wird als Trägerpolymer mindestens ein Polyacrylat und/oder mindestens ein hyperverzweigtes Polymer eingesetzt, wobei hyperverzweigte Polymere mit einem hydrophilen Kern und hydrophoben Endgruppen besonders bevorzugt sind.

Die zuvor dargelegten niedermolekularen Substanzen können einzeln oder als Mischung von zwei, drei oder mehr eingesetzt werden. Hierbei können die Mischungen niedermolekulare Substanzen der gleichen Klasse oder von unterschiedlichen Klassen umfassen. So kann beispielsweise eine Kombination als niedermolekulare Substanz eine Mischung umfassen, die ein Vitamin und ein Geschmacksstoff aufweist.

Gemäß einem besonderen Aspekt der vorliegenden Erfindung umfasst das Präparat vorzugsweise nur geringe Mengen an Kreatin, Folsäure oder Tocopherol, falls diese Stoffe allein als zu verkapselnde Substanz eingesetzt werden. Besonders bevorzugt ist die Menge an Keratin, Folsäure oder Tocopherol auf 10 Gew.-%, besonders bevorzugt 5 Gew.-% beschränkt, bezogen auf das Gewicht des Präparats, falls das Präparat keine weiteren zu verkapselnden Substanzen aufweist. Präparate, die Kombinationen von Kreatin, Folsäure und/oder Tocopherol untereinander oder mit weiteren zuverkapselnden Substanzen aufweisen, sind jedoch bevorzugt, wobei diese Präparate auch mehr als 10 Gew.-% an Keratin, Folsäure oder Tocopherol aufweisen können. Insbesondere umfassen bevorzugte Mikropartikel keine Kombinationen von zu verkapselnder Substanz und Trägerpolymer, die mehr als 10 Gew.-% Kreatin, Folsäure und/ oder Tocopherol als zu verkapselnde Substanz und hyperverzweigte Trägerpolymere mit einem hydrophilen Polyesterkern und hydrophoben Endgruppen, die von Carbonsäuren mit 16 bis 22 Kohlenstoffatomen abgeleitet sind, aufweisen.

Die erfindungsgemäßen Mikropartikel können einen überraschend hohen Anteil an zu verkapselnder Substanz aufweisen. Gemäß einem besonderen Aspekt der vorliegenden Erfindung kann das Gewichtsverhältnis von Trägerpolymer zur zu verkapselnder Substanz vorzugsweise im Bereich von 40:1 bis 0,5:1, besonders bevorzugt im Bereich von 20:1 bis 2:1 liegen. Der Beladungsgrad kann vorzugsweise in einem Bereich von 1% bis 99%, besonders bevorzugt 5% bis 90% und ganz besonders 10 bis 30% liegen. Der Beladungsgrad errechnet sich aus dem Verhältnis des Gewichts der zu verkapselnden Substanz zu dem Gesamtgewicht des Mikropartikels.

Gemäß dem Verfahren der vorliegenden Erfindung wird eine Polymerschmelze hergestellt, die mindestens ein Trägerpolymer und mindestens eine zu verkapselnde Substanz umfasst. Die zu verkapselnde Substanz wird vorzugsweise fein in der Polymerschmelze verteilt. Hierzu können bekannte Vorrichtungen, wie zum Beispiel Rührwerke, die einen Rührkessel mit Propeller-, Scheiben-, Zahnscheiben-, Anker-, Wendel-, Blatt-, Schaufel-, Schrägblatt-, Kreuzblatt-, Schrauben-, MIG-, INTERMIG-, ULTRA-TURRAX, Schnecken-, Band-, Finger-, Korb-, Impeller-Rührer umfassen, sowie Dispergatoren und Homogenisatoren, die unter anderem mit Ultraschall arbeiten können, eingesetzt werden. Die Vorrichtungen können im Allgemeinen mindestens eine Welle aufweisen, an der wiederum vorzugsweise 1 bis 5 Rührelemente angebracht sein können.

Hierbei kann beispielsweise eine Lösung, eine Suspension oder eine Dispersion entstehen, wobei die Teilchengröße der verteilt vorliegenden Phase vorzugsweise höchstens 5000 $\mu$m, besonders bevorzugt höchstens 1000 $\mu$m beträgt, falls die zu verkapselnde Substanz partikelförmig vorliegt.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 10000 Umdrehungen pro Minute liegen.

Die Temperatur bei der die Polymerschmelze hergestellt wird, kann ebenfalls in einem weiten Bereich liegen, wobei diese unter anderem von der Verfestigungstemperatur des Trägerpolymers abhängig ist. Vorzugsweise liegt die Temperatur im Bereich von -20°C bis 250°C, besonders bevorzugt im Bereich von 0°C bis 200°C. Gemäß einem besonderen Aspekt der vorliegenden Erfindung wird die Temperatur beim Herstellen der Polymerschmelze 10°C bis 200°C, besonders bevorzugt im Bereich 15°C bis 150°C oberhalb der Verfestigungstemperatur des Trägerpolymers gewählt. Der beim Herstellen der Polymerschmelze eingesetzte Druck ist ebenfalls unkritisch, wobei dieser vielfach von der Art der zu verkapselnden Substanz und der Verfestigungstemperatur des Trägerpolymers abhängig ist. Beispielsweise kann der Druck im Bereich von 0,1 mbar bis 200bar, bevorzugt im Bereich von 10 mbar bis 100 bar gewählt werden.

Gemäß einem besonderen Aspekt kann die Polymerschmelze vorzugsweise 10 bis 99,99 Gew.-%, besonders bevorzugt 50 bis 99,00 Gew.-% und ganz besonders bevorzugt 70 bis 90 Gew.-% Trägerpolymer umfassen. Des Weiteren kann die Polymerschmelze vorzugsweise 0,01 Gew.-% bis 90 Gew.-%, vorzugsweise 1 Gew.-% bis 50 Gew.-% und ganz besonders bevorzugt 10 Gew.-% bis 30 Gew.-% zu verkapselnde Substanz umfassen.

Gemäß einem besonderen Aspekt der vorliegenden Erfindung wird der Polymerschmelze vorzugsweise kein Lösungsmittel, insbesondere kein organisches Lösungsmittel zugegeben, wobei besonders bevorzugte Polymerschmelzen kein Lösungsmittel umfassen. Als Lösungsmittel wird hierbei eine Substanz verstanden, in der das Trägerpolymer löslich ist und die während des Herstellungsprozesses abgetrennt werden muss, da diese Verbindung nicht in den Mikropartikeln enthalten sein sollte. In diesem Zusammenhang ist festzuhalten, dass viele der zuvor dargelegten zu verkapselnden Substanzen Eigenschaften eines Lösungsmittels haben können. Diese Substanzen sind jedoch ein gewollter Bestandteil der Mikrokapseln, so dass diese Verbindungen im Rahmen der vorliegenden Erfindung kein Lösungsmittel darstellen. Dementsprechend ist die Verwendung von Lösungsmittel zur Durchführung des Verfahrens nicht notwendig. Andererseits werden einige zu verkapselnde Substanzen in gelöster Form geliefert, wobei die hierfür verwendeten Lösungsmittel im Allgemeinen für die Verwendung der zu verkapselnden Substanz nicht kritisch sind, so dass diese beispielsweise gesundheitlich unbedenklich sind, falls die zu verkapselnde Substanz biologisch wirksam ist. Derartige Hilfsstoffe müssen nicht notwendig vor der Herstellung der Polymerschmelze abtrennt werden. Vielmehr können diese Hilfssubstanzen in die Polymerschmelze eingearbeitet werden.

Die zuvor beschriebene Polymerschmelze wird erfindungsgemäß in eine zweite flüssige Phase überführt, in der das Trägerpolymer schwer löslich ist und die eine Verfestigungstemperatur unterhalb der Verfestigungstemperatur des Trägerpolymers aufweist. Dementsprechend umfasst die zweite flüssige Phase ein oder mehrere Substanzen, die mit der Polymerschmelze unverträglich sind und die als Hauptbestandteil der kontinuierlichen Phase dienen. Falls das Trägerpolymer bzw. die Polymerschmelze hydrophil ist, ist dementsprechend die zweite flüssige Phase hydrophob. Falls das Trägerpolymer bzw. die Polymerschmelze hydrophob ist, ist dementsprechend die zweite flüssige Phase hydrophil.

Der Begriff "schwer löslich" bedeutet, dass die Löslichkeit des Trägerpolymers in der zweiten flüssigen Phase möglichst gering sein sollte. Die Löslichkeit ist vielfach von der Temperatur abhängig. Dementsprechend können die Dispergierbedingungen vielfach so gewählt werden, dass ein möglichst geringer Anteil des Trägerpolymers in der zweiten flüssigen Phase gelöst wird. Vorzugsweise weist das Trägerpolymer eine Löslichkeit nach der Kolbenmethode bei der Dispergiertemperatur von höchstens 20 Massenprozent, bevorzugt höchstens 10 Massenprozent in der zweiten flüssigen Phase auf. Gemäß einem besonderen Aspekt kann das Trägerpolymer vorzugsweise eine Löslichkeit nach der Kolbenmethode bei 40°C von höchstens 20 Massenprozent in der zweiten flüssigen Phase aufweisen.

Die zweite flüssige Phase weist eine Verfestigungstemperatur unterhalb der Verfestigungstemperatur des Trägerpolymers auf. Im Allgemeinen ergibt sich diese Temperatur aus der Schmelztemperatur oder der Glasübergangstemperatur des Hauptbestandteils der zweiten flüssigen Phase, wobei Gefrierpunktserniedrigungen durch Hilfs- oder Zusatzstoffe bzw. durch die Verwendung von Stoffgemischen auftreten können. Diese Größe kann aus DSC-Messungen erhalten werden, wobei die Schmelzpunkte bzw. Gefrierpunkte der üblichen Hauptbestandteile der zweiten flüssigen Phase in Nachschlagewerken aufgeführt sind.

Zu den hydrophoben Substanzen, die als Hauptbestandteil in der zweiten flüssigen Phase enthalten sein können, gehören insbesondere Kohlenwasserstoffverbindungen, wie beispielsweise aromatische Substanzen, wie Toluol, Benzol und Xylol, gesättigte Kohlenwasserstoffe, wie beispielsweise Hexan, Cyclohexan, Heptan, Octan, Nonan, Decan, Dodecan, die auch verzweigt vorliegen können.

Zu den hydrophilen Substanzen, die als Hauptbestandteil in der zweiten flüssigen Phase enthalten sein können, gehören insbesondere Wasser und Alkohole mit 1 bis 7, vorzugsweise 1 bis 4 Kohlenstoffatomen, insbesondere Methanol, Ethanol, Propanol und/oder Butanol.

Die zweite flüssige Phase kann neben dem Hauptbestandteil zusätzliche Hilfsstoffe, insbesondere Dispergatoren und Stabilisatoren umfassen. Diese Hilfsstoffe sind in der Fachwelt bekannt, wobei Dispergatoren einer Aggregation der Partikel entgegenwirken. Hierzu gehören insbesondere Emulgatoren, Schutzkolloide und Tenside, die jeweils entsprechend den eingesetzten Trägerpolymeren, zu verkapselnder Substanz und Hauptbestandteil der zweiten flüssigen Phase eingesetzt werden können. Zu den bevorzugten Tensiden gehören insbesondere anionische Tenside, wie Laurylethersulfat, kationische Tenside und nichtionische Tenside, wie beispielsweise Polyvinylalkohole und ethoxylierte Fettalkohole. Stabilisatoren können für eine Vielzahl von Anwendungen eingesetzt werden, wobei diese Hilfsstoffe einen erwünschten, instabilen Zustand erhalten bzw. stabilisieren. Hierzu gehören insbesondere Absetzverhinderungsmittel, wie Pektine und/oder Karragenan.

Bevorzugt umfasst die zweite flüssige Phase 60 bis 100 Gew.-% Hauptbestandteil, beispielsweise die zuvor dargelegten hydrophilen Substanzen, wie Wasser oder Alkohole mit bis zu 4 Kohlenstoffatomen oder die zuvor dargelegten hydrophoben Substanzen. Weiterhin kann die zweite flüssige Phase 0 bis 40 Gew.-% Hilfssubstanzen enthalten, insbesondere 0 bis 20 Gew.-% Emulgatoren und 0 bis 20 Gew.-% Stabilisatoren.

Die in die zweite flüssige Phase eingebrachte Polymerschmelze wird bei einer Temperatur, die größer oder gleich der Verfestigungstemperatur des Trägerpolymers ist, dispergiert.

Die Verfestigungstemperatur des Trägerpolymers bezeichnet im Rahmen der vorliegenden Erfindung die Temperatur bei der das Trägerpolymer fest wird, so dass Polymerpartikel bei dieser Temperatur nicht mehr ohne äußere Einwirkungen zu größeren Aggregaten agglomerieren. Je nach Aufbau und Kristallisationseigenschaften kann die Verfestigungstemperatur beispielsweise durch die Glasübergangstemperatur bzw. die Schmelztemperatur des Trägerpolymers gegeben sein, die beispielsweise durch DSC-Verfahren (Differential Scanning Calorimetry; Dynamische Differenzkalorimetrie) bestimmt werden können. Hierbei ist festzuhalten, dass amorphe Polymere im Allgemeinen lediglich eine Glasübergangstemperatur aufweisen, wohingegen kristalline Polymere eine Schmelztemperatur zeigen. Teilkristalline Polymere können sowohl eine Glasübergangstemperatur als auch eine Schmelztemperatur zeigen, wobei in diesem Fall die Temperatur ausschlaggebend ist, bei der die Partikel keine Agglomeration zeigen. Falls die Oberfläche im Wesentlichen kristallin ist, so ist der Schmelzpunkt dieser Bestandteile entscheidend.

Dispergieren bedeutet in diesem Zusammenhang, dass die mindestens eine zu verkapselnde Substanz umfassende Polymerschmelze fein in der kontinuierlichen zweiten flüssigen Phase verteilt wird. Das Dispergieren kann hierbei mit bekannten Geräten und Vorrichtungen, wie zum Beispiel Rührwerken, die einen Rührkessel mit Propeller-, Scheiben-, Zahnscheiben-, Anker-, Wendel-, Blatt-, Schaufel-, Schrägblatt-, Kreuzblatt-, Schrauben-, MIG-, INTERMIG-, ULTRA-TURRAX, Schnecken-, Band-, Finger-, Korb-, Impeller-Rührer umfassen sowie Dispergatoren und Homogenisatoren, die unter anderem mit Ultraschall arbeiten können, durchgeführt werden. Die Vorrichtungen können im Allgemeinen mindestens eine Welle aufweisen, an der wiederum vorzugsweise 1 bis 5 Rührelemente angebracht sein können.

Das Verhältnis von Rührerdurchmesser zum Durchmesser des Rührkessels liegt vorzugsweise im Bereich von 0,05 bis 0,95, besonders bevorzugt 0,1 bis 0,9, wobei besonders bevorzugte Bereiche von der Art des Rührers abhängig sind. Bei Rührern mit einer sehr starken Scherwirkung (high shear), wie zum Beispiel ULTRA-TURRAX-Rührern, liegt das Verhältnis von Rührerdurchmesser zum Durchmesser des Rührkessels vorzugsweise im Bereich von 0,3 bis 0,7 besonders bevorzugt im Bereich von 0,4 bis 0,6. Bei Rührern mit einem Rührblatt, wie zum Beispiel Blatt-, Schaufel-, Schrägblattrührer, oder bei Korb-, Scheiben- oder Zahnscheibenrühren, liegt das Verhältnis von Rührerdurchmesser zum Durchmesser des Rührkessels vorzugsweise im Bereich von 0,1 bis 0,6 besonders bevorzugt im Bereich von 0,2 bis 0,4. Bei Verwendung von Propeller-, Schrauben-, Kreuzblatt-, MIG-, INTERMIG-, Schnecken- oder Impeller-Rührer liegt das Verhältnis von Rührerdurchmesser zum Durchmesser des Rührkessels vorzugsweise im Bereich von 0,3 bis 0,8 besonders bevorzugt im Bereich von 0,4 bis 0,7. Bei Verwendung von Anker-, Finger-, Band- oder Wendel-Rührerern liegt das Verhältnis von Rührerdurchmesser zum Durchmesser des Rührkessels vorzugsweise im Bereich von 0,8 bis 0,99, besonders bevorzugt 0,9 bis 0, 95. Diese Werte gelten insbesondere für einen bevorzugt mittigen Einbau des Rührers in dem Rührkessel. Bei einem Einbau am Rand des Rührkessels gelten leicht andere Werte, die der Fachmann leicht auffinden kann.

Vorzugsweise liegt das Längenverhältnis von Abstand des Rührers von dem Boden des Rührkessels zur Höhe des Rührkessels im Bereich von 0,1 bis 0,9, besonders bevorzugt 0,2 bis 0,8 und ganz besonders bevorzugt 0,4 bis 0,6, ohne dass hierdurch eine Beschränkung der Erfindung erfolgen soll.

Die Dauer und der Energieeintrag des Dispergierens sind hierbei von der gewünschten Partikelgröße und Partikelgrößenverteilung abhängig. Dementsprechend kann die Dauer des Dispergierens in einem weiten Bereich gewählt werden. Vorzugsweise wird das Dispergieren für eine Zeitdauer im Bereich von 1 Sekunde bis 5 Stunden, besonders bevorzugt im Bereich von 10 Sekunden bis 2 Stunden durchgeführt.

Überraschend gelingt es bei Verwendung der zuvor dargelegten Rührparameter eine geringe Partikelgröße und enge Partikelgrößenverteilung bei einer Dispergierzeit im Bereich von 2 Sekunden bis 45 Minuten, besonders bevorzugt 5 Sekunden bis 60 Sekunden zu erzielen.

Gemäß einem besonderen Aspekt der vorliegenden Erfindung kann die Newtonzahl beim Dispergieren vorzugsweise im Bereich von 0,1 bis 1000, besonders bevorzugt im Bereich von 0,4 bis 800 liegen.

Die Newtonzahl errechnet sich im Allgemeinen aus der Formel

$$N_{Po} = P / (\rho \cdot n^3 \cdot d^5)$$

wobei

P die Rührleistung [W]
d der Durchmesser des Rührers [m]
ρ die Dichte der Flüssigkeit in dem System [kg.m$^{-3}$]
n die Frequenz bzw. die Rotationsgeschwindigkeit [s$^{-1}$]

bedeutet.

Entsprechend einer besonderen Ausführungsform des erfindungsgemäßen Verfahrens kann die Reynoldszahl beim Dispergieren vorzugsweise im Bereich von 1 bis $10^7$, besonders bevorzugt im Bereich von 10 bis $10^6$ liegen.

Die Reynoldszahl ist an sich bekannt und berechnet sich bei Strömungen in Röhren aus der folgenden Formel:

$$N_{Re} = U \cdot L \cdot \rho / \mu$$

wobei

U die mittlere lineare Geschwindigkeit der Flüssigkeit [m·s$^{-1}$],
L die charakteristische Länge des Systems [m],
ρ die Dichte der Flüssigkeit in dem System [kg.m$^{-3}$]
µ die dynamische Viskosität der Flüssigkeit in dem System [kg.m$^{-1}$s$^{-1}$]

und
bedeutet.

In Reaktoren wird üblich die Umlaufgeschwindigkeit herangezogen, so dass folgende Formel Verwendung findet:

$$N_{Re} = n \cdot L^2 \cdot \rho / \mu$$

oder

$$N_{Re} = n \cdot L^2 / \nu$$

wobei

n die Frequenz bzw. die Rotationsgeschwindigkeit [s$^{-1}$],
ν die kinematische Viskosität, die µ/ρ ist [m$^2$.S$^{-1}$],
L die charakteristische Länge des Systems [m],
ρ die Dichte der Flüssigkeit in dem System [kg.m$^{-3}$],
µ die dynamische Viskosität der Flüssigkeit in dem System [Kg.m$^{-1}$s$^{-1}$]

bedeutet.

Die hierzu notwendigen Parameter sind von den zuvor dargelegten Vorrichtungen abhängig. Vorzugsweise kann die Rührgeschwindigkeit im Bereich von 10 bis 25000 Umdrehungen pro Minute, besonders bevorzugt im Bereich von 20 bis 10000 Umdrehungen pro Minute liegen.

Hierbei ist die eingesetzte Newtonzahl bzw. die Rührgeschwindigkeit von der gewünschten Partikelgröße und Partikelgrößenverteilung abhängig. Je mehr Energie zugeführt wird und je länger dispergiert wird, desto kleinere Partikelgrößen können erzielt werden. Eine enge Partikelgrößenverteilung kann ebenfalls durch eine hohe Dispergierenergie und eine lange Dispergierzeit erzielt werden. Andererseits sind lange Dispergierzeiten

und hohe Dispergierenergien vielfach mit zusätzlichen Kosten verbunden.

Die Temperatur bei der die Polymerschmelze in der zweiten flüssigen Phase dispergiert wird, kann ebenfalls in einem weiten Bereich liegen, wobei diese unter anderem von der Verfestigungstemperatur des Trägerpolymers abhängig ist. Vorzugsweise liegt die Temperatur im Bereich von -20°C bis 250°C, besonders bevorzugt im Bereich von 0 bis 200°C. Der beim Dispergierten der Polymerschmelze eingesetzte Druck ist ebenfalls unkritisch, wobei dieser vielfach von der Art der zu verkapselnden Substanz und der Verfestigungstemperatur des Trägerpolymers abhängig ist. Beispielsweise kann der Druck im Bereich von 10 mbar bis 200 bar, bevorzugt im Bereich von 100 mbar bis 100 bar gewählt werden.

Die Temperatur beim Dispergieren ist größer oder gleich der Verfestigungstemperatur des Trägerpolymers. Vorzugsweise liegt die Dispergiertemperatur 1°C bis 200°C, besonders bevorzugt 5°C bis 150°C und ganz besonders bevorzugt 10 bis 50°C oberhalb der Verfestigungstemperatur des Trägerpolymers.

Das Gewichtsverhältnis von Polymerschmelze zur zweite flüssigen Phase kann in einem weiten Bereich liegen. Vorzugsweise liegt dieses Verhältnis im Bereich von 1:1 bis 1:1000, besonders bevorzugt 1:1,5 bis 1:500.

Beim Dispergieren kann die Zusammensetzung beispielsweise 50 bis 99 Gew.-%, bevorzugt 70 bis 98 Gew.-% zweite flüssige Phase und 1 bis 50 Gew.-%, bevorzugt 2 bis 30 Gew.-% Polymerschmelze umfassen.

Nachdem die Polymerschmelze in der zweiten flüssigen Phase dispergiert vorliegt, wird die dispergierte Polymerschmelze durch Abkühlen der zweiten flüssigen Phase auf eine Temperatur unterhalb der Verfestigungstemperatur des Trägerpolymers verfestigt.

Die Art der Abkühlung ist unter anderem von der gewünschten Partikelgröße und Partikelgrößenverteilung abhängig. Eine schnelle Abkühlung kann unter anderem zu einer besonders einheitlichen Partikelgrößenverteilung und kleinen Partikeln führen, da eine Aggregation vermieden werden kann. Hierbei ist die Bildung von Agglomeraten bei einem großen Abkühlvolumen geringer.

Des Weiteren können die Partikelgrößenverteilung und die Größe der Partikel über Hilfsmittel, wie beispielsweise Dispergatoren und Emulgatoren, beeinflusst werden. Diese Additive können beispielsweise in die zweite Phase hinzu gegeben werden, wobei eine Additivierung der Oberfläche der gebildeten Partikel erzielt werden kann. Diese Additivierung kann auch eine Aggregation der Mikropartikel während des Trocknens oder beim Lagern vermindern.

In einer bevorzugten Ausführungsform ist zwischen dem Dispergieren der Polymerschmelze und dem Verfestigen eine Verweilzeit vorgesehen, in der die Polymerschmelze gezielt aggregiert wird, um den Anteil an kleinen Partikeln zu verringern. Überraschend kann hierdurch der Anteil an Partikeln mit einer geringen Größen, vorzugsweise im Bereich unterhalb von 0,1 µm, besonders bevorzugt unterhalb von 1 µm und ganz besonders bevorzugt unterhalb von 5 µm stark verringert werden, so dass hierdurch besonders enge Partikelverteilungen erhalten werden können. Hierbei liegt der Anteil an Emulgatoren vorzugsweise in einem Bereich, der eine Aggregation ermöglicht. Vorzugsweise liegt die Verweilzeit in einem Bereich von 0,5 Sekunden bis 1 Stunde, besonders bevorzugt 1 Sekunde bis 10 Minuten.

Je nach Anwendung kann die so erhaltene Zusammensetzung unmittelbar weiter verarbeitet werden, ohne dass eine Aufreinigung, Aufkonzentration oder Abtrennung vorgenommen wird. Gemäß einer besonderen Ausführungsform kann das vorliegende Verfahren den Schritt des Abtrennens der in der zweiten flüssigen Phase gebildeten Mikropartikel aufweisen. Das Abtrennen kann durch bekannte Verfahren, insbesondere durch Filtrieren, Zentrifugieren, Sedimentation, Magnettrennung, Flotation, Sieben oder Dekantieren erfolgen, wobei die Verfahren einzeln oder in Kombination eingesetzt werden können. Hierbei können die Verbindungen der zweiten flüssigen Phase im wesentlichen vollständig abgetrennt werden, so dass getrocknete Mikropartikel erhalten werden oder die Partikel können aufkonzentriert werden, wobei noch größere Mengen an Verbindungen der zweiten flüssigen Phase im Endprodukt enthalten sind. Vorzugsweise kann das Endprodukt mindestens 80 Gew.-% Mikropartikel, besonders bevorzugt mindesten 95 Gew.-% Mikropartikel aufweisen.

Die zum Abtrennen oder Aufkonzentrieren der Mikropartikel verwendbaren Vorrichtungen, nachfolgend auch Separatoren genannt, sind allgemein bekannt. So können unter anderem Zentrifugen, Dekanter, Fliehkraftabscheider, Filter, beispielsweise Schwerkraftfilter, Saugfilter (Vakuumfilter), Druckfilter, Saug-Druck-Filter, Pressfilter, Vakuum-Trommelfilter, Bandfilter, Scheibenfilter, Planfilter, Kammerfilterpresse, Rahmenfilterpresse, Kerzenfilter, Blattfilter, Membranfilterplatte und/oder Siebbandpressen eingesetzt werden.

Die Temperatur beim Abtrennen oder Aufkonzentrieren kann ebenfalls in einem weiten Bereich liegen, wobei diese unter anderem von der Verfestigungstemperatur des Trägerpolymers abhängig ist. Um eine Aggregation der Partikel zu vermeiden, sollte die gewählte Temperatur unterhalb der Verfestigungstemperatur des Trägerpolymers liegen. Vorzugsweise liegt die Temperatur im Bereich von -20°C bis 250°C, besonders bevorzugt im Bereich von -10°C bis 100°C. Der beim Abtrennen oder Aufkonzentrieren eingesetzte Druck ist ebenfalls unkritisch, wobei dieser vielfach von der Art der zu verkapselnden Substanz und der Verfestigungstemperatur des Trägerpolymers abhängig ist. Beispielsweise kann der Druck im Bereich von 10 mbar bis 200 bar, bevorzugt im Bereich von 100 mbar bis 100 bar gewählt werden.

Nach dem Abtrennen können die erhaltenen Partikel gewaschen werden. Hierzu können die Partikel mit einer

Waschflüssigkeit behandelt werden, um Additivreste und/oder zu verkapselnde Substanz, die sich auf der Oberfläche der Partikel befindet von den Partikeln zu trennen. Dementsprechend sollten die Partikel, insbesondere die Trägerpolymere nicht in der Waschflüssigkeit löslich sein. Andererseits sollte die abzutrennende Substanz, beispielsweise die zu verkapselnde Substanz eine möglichst hohe Löslichkeit aufweisen. Dementsprechend können die Waschflüssigkeiten hydrophob oder hydrophil sein, je nach Art des Trägerpolymeren. Zu den bevorzugten hydrophoben Waschflüssigkeiten zählen insbesondere Kohlenwasserstoffverbindungen, wie beispielsweise aromatische Substanzen, wie Toluol, Benzol und Xylol, gesättigte Kohlenwasserstoffe, wie beispielsweise Cyclohexan, Heptan, Octan, Nonan, Decan, Dodecan, die auch verzweigt vorliegen können. Zu den bevorzugten hydrophilen Waschflüssigkeiten zählen insbesondere Wasser und/oder Alkohole mit 1 bis 7, vorzugsweise 1 bis 4 Kohlenstoffatomen, insbesondere Methanol, Ethanol, Propanol und/oder Butanol. Diese Flüssigkeiten können einzeln oder auch als Mischung von zwei, drei oder mehr Flüssigkeiten eingesetzt werden.

Die Temperatur beim Waschen kann ebenfalls in einem weiten Bereich liegen, wobei diese unter anderem von der Verfestigungstemperatur des Trägerpolymers abhängig ist. Um eine Aggregation der Partikel zu vermeiden, sollte die gewählte Temperatur unterhalb der Verfestigungstemperatur des Trägerpolymers liegen. Vorzugsweise liegt die Temperatur im Bereich von -20°C bis 250°C, besonders bevorzugt im Bereich von -10 °C bis 100 °C. Der beim Waschen eingesetzte Druck ist ebenfalls unkritisch, wobei dieser vielfach von der Art der zu verkapselnden Substanz und der Verfestigungstemperatur des Trägerpolymers abhängig ist. Beispielsweise kann der Druck beim Waschen im Bereich von 10 mbar bis 200 bar, bevorzugt im Bereich von 100 mbar bis 100 bar gewählt werden.

Die zum Waschen der Partikel verwendbaren Vorrichtungen sind allgemein bekannt. So können hierfür beispielsweise Vorrichtungen eingesetzt werden, die ein Mischgefäß und einen Separator umfassen. Die Mischgefäße umfassen vorzugsweise die zuvor dargelegten Geräte und Vorrichtungen zum Dispergieren.

In einem weiteren Schritt können die erhaltenen Mikropartikel getrocknet werden. Die zum Trocknen der Mikropartikel verwendbaren Vorrichtungen sind allgemein bekannt. So können unter anderem Trommeltrockner, Taumeltrockner, Tellertrockner, Schneckentrockner, Schaufeltrockner, Zylindertrockner, Walzentrockner, Gefriertrockner, Wirbelschichttrockner, Sprühtrockner, Stromtrockner, Mahltrockner, Hordentrockner, Tunneltrockner, Vakuumtrockner- und/oder Vakuumkontakttrockner eingesetzt werden.

Die Temperatur beim Trocknen kann ebenfalls in einem weiten Bereich liegen, wobei diese unter anderem von der Verfestigungstemperatur des Trägerpolymers abhängig ist. Um eine Aggregation der Partikel zu vermeiden, sollte die gewählte Temperatur unterhalb der Verfestigungstemperatur des Trägerpolymers liegen. Vorzugsweise liegt die Temperatur beim Trocknen im Bereich von -20°C bis 150°C, besonders bevorzugt im Bereich von -10 °C bis 100 °C. Der beim Trocknen eingesetzte Druck ist ebenfalls unkritisch, wobei dieser vielfach von der Art der zu verkapselnden Substanz und der Verfestigüngstemperatur des Trägerpolymers abhängig ist. Beispielsweise kann der Druck im Bereich von 0,1 mbar bis 10 bar, bevorzugt im Bereich von 0,2 mbar bis 2 bar gewählt werden.

Das zuvor beschriebene Verfahren kann mit einfachen Anlagen durchgeführt werden, die aus an sich bekannten Komponenten aufgebaut sein können. Geeignete Anlagen umfassen mindestens drei Mischgefäße, einen Separator, eine Zuführung und ein Mischventil, wobei mindestens zwei Mischgefäße über Zuführungen mit einem dritten Mischgefäß verbunden sind, das dritte Mischgefäß mit dem Separator verbunden ist und das Mischventil in der Zuführung zwischen dem Mischgefäß und dem Separator vorgesehen ist. Die im Separator abgetrennte zweite Phase kann vorzugsweise über eine Rückführung in ein Mischgefäß zurückgeführt werden.

Gemäß einer bevorzugten Ausführungsform kann in der Leitung zwischen dem ersten Mischgefäß, in dem die Polymerschmelze hergestellt wird und dem zweiten Mischgefäß, in dem die Polymerschmelze in der zweiten flüssigen Phase dispergiert wird, eine Pumpe vorgesehen sein, die für hochviskose Flüssigkeiten geeignet ist. Pumpen und Förderaggregate, die zur Förderung viskoser und hochviskoser Medien geeignet sind, können der Fachliteratur, insbesondere Kapitel P und R in Dubbel "Taschenbuch für den Maschinenbau", 19.Auflage (1997) Springer-Verlag Berlin Heidelberg New York, entnommen werden. Zu den bevorzugten Pumpen gehören insbesondere Schraubenpumpen, beispielsweise Schraubenpumpen mit einer, zwei oder drei Schrauben, Schraubenverdichter, Flügelpumpen, Drehkolbenpumpen, Rotationspumpen, Kolbenpumpen, Rotationskolbenpumpen und/oder Schlauchpumpen.

Hierbei können diese Pumpen unmittelbar in den Leitungen vorgesehen sein, über die die verschiedenen Vorrichtungen bzw. Mischgefäße miteinander verbunden sind. Gemäß besonders zweckmäßigen Abwandlungen können auch außerhalb dieser Verbindungsleitungen Pumpen angeordnet sein, um vorzugsweise über einen Injektionsstrom einen Transport zwischen den verschiedenen Vorrichtungen bzw. Mischgefäßen zu bewirken. Zweckmäßig kann beispielsweise ein Kühlstrom über eine Pumpe mit Druck als Injektorstrom in ein Mischventil eingeleitet werden. Der hierdurch im Mischventil entstehende Unterdruck kann eingesetzt werden, um aus dem Mischgefäß zur Herstellung der Dispersion die zu verfestigende, dispergierte Polymerschmelze samt kontinuierlicher Phase anzusaugen und, nach dem Verfestigen im Mischventil, in einen Separator zu überführen. Unter dem Begriff "Mischventil" wird im Rahmen der Beschreibung und der Ansprüche ein Bauteil oder Apparat verstanden, in dem zwei oder mehrere Zulaufströme in Kontakt gebracht werden mit dem Ziel, diese beiden Ströme zu mischen. Zur Kontrolle des Mischvorgangs sind in den Zuläufen und gegebenenfalls auch im Ablauf Steuerelemente vorzusehen, die eine diskrete oder kontinuierliche Einstellung oder Regelung des Massenstroms zulassen. Diese Steuerelemente können manuell, halbautomatisch oder automatisch betätigt werden. Bevorzugte Ausführungsformen eines Mischventils sind ein einfaches T-Stück, dessen Zulaufströme über Ventile eingestellt werden können, oder das oben beschriebene Injektorventil.

Gemäß einem besonderen Aspekt der vorliegenden Erfindung weist die Anlage vorzugsweise mindestens drei Mischgefäße auf, wobei mindestens zwei Mischgefäße über Zuführungen mit mindestens einem Mischgefäß verbunden sind. Hierbei dient mindestens ein Mischgefäß zum Herstellen der Polymerschmelze, mindestens ein Mischgefäß zum Herstellen der zweiten flüssigen Phase und mindestens ein Mischgefäß zum Dispergieren der Polymerschmelze in der zweiten flüssigen Phase. Die Polymerschmelze und die zweite flüssige Phase können in weiteren separaten Mischgefäßen chargenweise oder kontinuierlich hergestellt werden, um eine kontinuierliche Produktion von Mikropartikeln sicherzustellen.

Die in der Anlage zur Herstellung von erfindungsgemäßen Mikropartikeln eingesetzten Mischgefäße können temperierbar ausgestattet sein. Dementsprechend können diese Mischgefäße Heizelemente oder Kühlelemente umfassen.

Vorzugsweise kann die Anlage mindestens einen Trockner aufweisen, der mit dem Separator verbunden ist. Des Weiteren kann die Anlage bevorzugt eine Vorrichtung zum Waschen von Partikeln umfassen.

Im vorliegenden Verfahren wird zum Verfestigen der in der zweiten flüssigen Phase dispergierten Polymerschmelze ein Mischventil eingesetzt, dem eine Kühlflüssigkeit und das Produkt aus Schritt c), d.h. die in der flüssigen Phase dispergierte Polymerschmelze zugeführt wird. Die Kühlflüssigkeit wird dabei über eine Zuleitung zugeführt, die einen größeren Durchmesser aufweist als die Zuleitung, über die das Produkt aus Schritt c) in das Mischventil eingeleitet wird. Durch diese Ausgestaltung ist es möglich, dass die Polymerschmelze mit einer hohen Geschwindigkeit in ein großes Volumen an Kühlflüssigkeit gegeben wird, wodurch eine sehr schnelle Abkühlung erzielt wird, ohne dass eine Aggregation der Teilchen eintritt. Vorzugsweise ist das Verhältnis der Geschwindigkeit, mit der das Produkt aus Schritt c) in das Mischventil eingeleitet wird, zur Geschwindigkeit der Kühlflüssigkeit größer als 1, besonders bevorzugt größer als 2. Besonders bevorzugt liegt das Verhältnis im Bereich von 1 bis 30, insbesondere im Bereich von 3 bis 10. Die Geschwindigkeit berechnet sich hierbei aus dem Volumen der abzukühlenden Phase, die neben der Polymerschmelze die zweite flüssige Phase umfasst. Vorzugsweise liegt das Volumenverhältnis von Kühlflüssigkeit zu dem Produkt aus Schritt c) im Bereich von 20:1 bis 1:20, besonders bevorzugt 10:1 bis 1:1. Die Ausführungsform des Verfahrens mit einem Mischventil ermöglicht eine schnelle, gezielte Verfestigung der Polymerschmelze und liefert überraschend besonders gleichmäßige Partikel.

In einer weiteren bevorzugten Ausführungsform der vorliegenden Erfindung wird die Leitungslänge, die zwischen dem Mischgefäß zum Dispergieren der Polymerschmelze in der zweiten flüssigen Phase und der Vorrichtung in der bzw. über die die Polymerschmelze verfestigt wird, so gewählt, dass in der Leitung eine gezielte Aggregation der kleinen Partikel erfolgt. Vorzugsweise liegt diese Länge im Bereich von 0,1 m bis 100 m, besonders bevorzugt 1 m bis 10 m.

Die Anlage kann Pumpen umfassen, die beispielsweise für den Transport von Flüssigkeiten oder zum Erzeugen von Über- bzw. Unterdruck dienen können. Geeignete Pumpen sind vom jeweiligen Zweck abhängig. Zu den bevorzugten Pumpen gehören beispielsweise Verdrängerpumpen, wie zum Beispiel Schöpfwerke, Förderschnecken, Balgpumpen, Kolbenpumpen, Rotationskolbenpumpen, Zahnradpumpen außen/innenverzahnt, Membranpumpen, Drehschieberpumpen, Zentrifugalpumpe, Schlauchpumpen, Zahnriemenpumpen, Exzenterschneckenpumpen, Schraubenpumpen und Schraubenverdichter und/oder Hydraulischer Widder; Strömungspumpen, wie zum Beispiel Kreiselpumpe, Axialpumpe, Diagonalpumpe und/oder Radialpumpe; Blasenpumpe, Wasserstrahlpumpe, Dampfstrahlpumpe, Stoßheber (Hydraulischer Widder), Pferdekopfpumpe (Tiefpumpe); Vakuumpumpen, wie zum Beispiel Verdrängerpumpe, Treibmittelpumpe, Molekularpumpe, Turbomolekularpumpe, Kryopumpe, Sorptionspumpe, Öldiffusionspumpe.

Derartige Anlagen werden in den nachfolgend näher beschriebenen Figuren beispielhaft erläutert.

Figur 1 beschreibt eine Ausführungsform einer Anlage zur Durchführung des Verfahrens der vorliegenden Erfindung.

Figur 1a beschreibt eine spezifische Abwandlung der Anlage zur Durchführung des Verfahrens der vorliegenden Erfindung gemäß Figur 1.

Figur 1b beschreibt eine spezifische Abwandlung der Anlage zur Durchführung des Verfahrens der vorliegenden Erfindung gemäß Figur 1.

In Figur 1 ist eine Ausführungsform einer Anlage zur Durchführung des Verfahrens der vorliegenden Erfindung dargestellt.

Die Ausführungsform weist ein Mischgefäß 3 mit Zuführungen 1, 2 zur Herstellung einer Polymerschmelze, ein Mischgefäß 6 zum Dispergieren der Polymerschmelze in einer zweiten flüssigen Phase, einen Separator 13 und einen Trockner 17 auf.

Diese Anlage weist beispielsweise ein, zwei oder mehr Zuführungen 1 bzw. 2, beispielsweise Leitungen oder Zuführschnecken, auf, über die ein oder mehrere Trägerpolymere und/oder ein oder mehrere zu verkapselnde Substanzen einem ersten Mischgefäß 3 zugeführt werden. Im Mischgefäß 3 können die zugeführten Substanzen in eine Polymerschmelze überführt werden, die mindestens ein Trägerpolymer und mindestens eine zu verkapselnde Substanz umfasst. In Mischgemäß 3 können die Komponenten fein ineinander verteilt werden. So kann beispielsweise eine Lösung, eine Dispersion oder Suspension hergestellt werden. Vielfach bildet das Trägerpolymer die Matrixphase, in der die zu verkapselnde Substanz verteilt wird. Hierfür können die zuvor beschriebenen Vorrichtungen eingesetzt werden.

Die zweite flüssige Phase wird in einem weiteren Mischgefäß 19. hergestellt, das beispielsweise ein, zwei, drei oder mehr Zuführungen 20, 21, 22 aufweisen kann. Über die Zuführungen 20, 21 und 22 können die Komponenten der zweiten flüssigen Phase, als Hauptbestandteil beispielsweise Wasser oder Ethanol sowie Hilfsstoffe, beispielsweise Stabilisatoren und Emulgatoren in das Mischgefäß 19 zugegeben werden. Das Wasser oder das Ethanol kann unabhängig von den weiteren Komponenten durch jede der zuvor dargelegten Verbindungen der zweiten flüssigen Phase ersetzt werden.

Die in Mischgefäß 19 erhaltene Lösung kann beispielsweise mit einer Pumpe 23 über die Zuführung 9, beispielsweise eine Leitung, in das Mischgefäß 6 überführt werden. In Mischgefäß 6 wird die Polymerschmelze in der zuvor beschriebenen Lösung dispergiert. Hierfür weist das Mischgefäß 6 bekannte Vorrichten zum Dispergieren auf. Hierfür können die zuvor beschriebenen Vorrichtungen eingesetzt werden.

Nachdem durch das Dispergieren die gewünschte Tropfengröße und Tropfengrößenverteilung erhalten wurde, kann die in der zweiten flüssigen Phase, beispielsweise Wasser, dispergiert vorliegende Polymerschmelze verfestigt werden. Die Abkühlung der Polymerschmelze nach dem Dispergieren in der zweiten flüssigen Phase wird durch das Zuleiten einer kalten Flüssigkeit über Leitung 25 erzielt, die im Wesentlichen der Zusammensetzung der zweiten flüssigen Phase entspricht, um die Polymerschmelze zu verfestigen. Die Zuleitung der kalten Flüssigkeit erfolgt über ein Mischventil 26, das in Leitung 12 zwischen dem Mischgefäß 6 und dem Separator 13 vorgesehen ist.

Über die Rückführung 14 kann ein Teil der im Separator 13 abgetrennten zweiten flüssigen Phase, beispielsweise Wasser oder Ethanol, die zusätzlich Hilfsstoffe, wie Emulgatoren oder Stabilisatoren enthalten kann, in das Mischgefäß 6 zurückgeführt werden. Somit können von Mischgefäß 19 lediglich die Mengen an zweiter flüssiger Phase in das Mischgefäß 6 eingeleitet werden, die nicht im Separator 13 zurück gewonnen werden können. Hierbei kann dieser Teil auf die Temperatur des Mischgefäßes 6 erwärmt werden. Ein weiterer Teil der im Separator 13 abgetrennten zweiten flüssigen Phase kann in die Leitung 25 geleitet werden. Hierbei kann die zweite Phase abgekühlt werden, so dass die Temperatur der in die Leitung 25 eingeleitetem zweiten flüssigen Phase der Temperatur der kalten Flüssigkeit entspricht.

Durch diese besondere Ausgestaltung kann das vorliegende Verfahren auch kontinuierlich durchgeführt werden. Des Weiteren kann diese Ausführungsform besonders energiesparend betrieben werden.

In einer besonderen Ausführungsform wird die Pumpe 11 durch einen Injektionsstrom ersetzt. Dies ist in den Abbildungen 1a und 1b gezeigt. In dieser Ausführungsform ist eine Pumpe außerhalb des Dispersionsstromes angeordnet. Beispielsweise kann eine Pumpe 25a in Leitung 25 vorgesehen sein, mit der der Strom der Kühlflüssigkeit in das Mischventil 26 gepumpt wird, wie dies in Figur 1a dargestellt ist. Bei der Ausführung des Mischventils als Injektor ist die Geometrie so zu gestalten, dass der eintretende Kühlstrom einen Unterdruck erzeugt, mit dem die Dispersion angesaugt wird.

In einer weiteren Abwandlung kann weitere Flüssigkeit in einem über eine Leitung 25b in einem Nebenstrom eingeleitet werden, um einen entsprechenden Unterdruck zu erzeugen, der eine Mischung der Kühlflüssigkeit mit der Polymerschmelze in Mischventil 26a bewirkt. Hierbei kann eine zweistufige Abkühlung erzielt werden, da in Leitung 25 weitere Kühlflüssigkeit zugeleitet werden kann, wobei diese Ausführungsform zwei Mischventile 26 und 26a umfasst. Diese Ausführungsform ist in Abbildung 1b dargestellt. Durch diese Anordnung können überraschende Vorteile erzielt werden. Insbesondere gelingt es hierdurch, besonders gleichmäßige, sphärische Partikel zu erhalten. Diese zweistufige Abkühlung wird vorzugsweise angewendet, wenn das Verhältnis von Kühlflüssigkeit und Dispersion in Mischventil 26a zur Einstellung einer bestimmten Partikelgröße festgelegt ist oder der zur Erzeugung des Unterdrucks notwendige Injektorimpuls zu einer Deformation der Polymerschmelze vor der Verfestigung führen würde. Vorzugsweise wird das verfahren dann so ausgeführt, dass die dispergierte Polymerschmelze in Figur 1b in Mischkammer 26 bereits verfestigt oder zumindest an der Oberfläche verfestigt vorliegt, um eine Deformation der Partikel zu vermeiden.

Die Druck- und Strömungsverhältnisse können über den Injektionsstrom und die Injektorgeometrie eingestellt werden. Diese Ausführungsformen ermöglichen überraschend einen besonders sicheren Betrieb der Anlage. In diesem Zusammenhang ist zu bedenken, dass bei einem Ausfall des Kühlmittelstromes ein weiteres Zulaufen des Emulsionsstromes verhindert werden kann. Weiterhin können mechanische Einflüsse der Pumpe auf die dispergierten, eventuell noch nicht vollständig verfestigten Polymerpartikel vermieden werden.

Die so erhaltenen Partikel können aus der zweiten flüssigen Phase abgetrennt werden. Hierzu wird die erhaltene Zusammensetzung, die verfestigte Mikropartikel aufweist, beispielsweise mit einer Pumpe 11 über die Leitung 12 in den Separator 13 überführt werden. Der

Separator 13 dient zum Abtrennen oder Aufkonzentrieren der in der zweiten flüssigen Phase enthaltenen Mikropartikel, wobei jede der zuvor dargelegten Vorrichtungen hierfür eingesetzt werden kann. Vorliegend werden im Separator die Mikropartikel von der zweiten flüssigen Phase getrennt, wobei vielfach eine Aufkonzentration ausreichend sein kann. Die abgetrennte zweite flüssige Phase, die beispielsweise Wasser, Emulgatoren und Stabilisatoren umfassen kann, kann über eine Rückführung 14, beispielsweise eine Leitung in das Mischgefäß 6 eingeleitet werden.

Die abgetrennten Mikropartikel können beispielsweise mit einer Pumpe 15 über die Zuführung 16, beispielsweise eine Leitung, in den Trockner 17 überführt werden. Im Trockner 17 können Reste der zweiten flüssigen Phase, beispielsweise Wasser entfernt werden. Die getrockneten Mikropartikel können über die Leitung 18 dem Trockner entnommen werden.

Eine weitere Ausführungsform weist eine Vorrichtung zum Waschen der Partikel auf.

Nachfolgend wird die vorliegende Erfindung durch Beispiele näher erläutert, ohne dass hierdurch eine Beschränkung erfolgen soll.

## Beispiele

### Beispiel 1

Unter Verwendung der in Abbildung 1 dargestellten Anlage, die ein Mischventil zum Verfestigen der Polymerschmelze aufwies, wurde ein erfindungsgemäßes Präparat hergestellt, welches Vitamin E (Tocopherol) und einen hyperverzweigten Polyester umfasste.

Der eingesetzte hyperverzweigte Polyester wurde durch Hydrophobisierung eines hydrophilen hyperverzweigten Polyesters hergestellt, der ein Gewichtsmittel des Molekulargewichts Mw von 3500 g/mol, eine Glastemperatur von etwa 35°C und eine Hydroxy-Zahl von etwa 490 mg KOH/g aufweist und kommerziell von der Firma Perstorp® unter der Bezeichnung Boltorn H30® erhältlich ist. Die Hydrophobisierung erfolgte durch Veresterung des hydrophilen Polymers mit einer Mischung aus Stearinsäure und Palmitinsäure (massenbezogenes Verhältnis Stearinsäure zu Palmitinsäure = 2 zu 1), wobei 80% der Hydroxygruppen des hydrophilen Polymers umgesetzt wurden. Das Molekulargewicht Mw betrug 10000 g/mol. Weitere Einzelheiten zur Herstellung des hydrophilen Polymers sowie zur Veresterung können den Schriften EP 0 630 389 B1 oder WO 93/01760 entnommen werden.

Das zur Herstellung der Polymerschmelze eingesetzte Mischgefäß hatte ein Volumen von ca. 1 1, wobei ein Ankerrührer zur Herstellung der Polymerschmelze eingesetzt wurde, der auf einer Höhe von 1/10 der Rührkesselhöhe oberhalb des Rührkesselbodens angeordnet wurde. Zur Herstellung des Präparates wurden 20 Gew. % Vitamin E (CAS: 101 91-41-0; kommerziell erhältlich von der Firma Sigma Aldrich) in dem geschmolzenen Polymer bei einer Temperatur von etwa 80°C mit einem Ankerrührer bei 200 Umdrehungen pro Minute in einem ersten Mischgefäß 5 Minuten dispergiert.

Das Mischgefäß zur Herstellung der kontinuierlichen Phase wies ein Volumen von ca. 6 1 auf und war mit einem Schrägblattrührer versehen, der auf einer Höhe von 1/3 der Rührkesselhöhe oberhalb des Rührkesselbodens eingerichtet wurde. Hierin wurde eine Lösung aus Tensiden bestehend aus 1 Gew. % Polyvinylalkohol (M = 6000 g/mol, Polisciences®, Warrington, USA) und 0,1 Gew. % eines ethoxylierten Fettalkohols (Tego® Alkanol L4 der Firma Degussa® AG) in Wasser bei 60°C unter Rühren mit 500 Umdrehungen pro Minute erzeugt.

Die in den zuvor dargelegten Mischgefäßen erhaltenen Zusammensetzungen wurden in ein drittes Mischgefäß überführt, um eine Dispersion (Emulsion) herzustellen. Das Mischgefäß zur Herstellung der Dispersion der Polymerschmelze in der kontinuierlichen Phase wies ein Volumen von ca. 0,5 l (Höhe ca. 0,1 m; Durchmesser ca. 0,05 m) auf, wobei als Rührer ein Ultra-Turrax UT25-Gerät in ca. halber Höhe des Mischgefäßes angeordnet wurde (0,05 m).

In dem dritten Mischgefäß wurden die beiden Phasen bei ca. 60°C dispergiert, wobei der ULTRA-TURRAX Rührer bei 8.000 Umdrehungen pro Minute betrieben wurde. Die Verweilzeit betrug ca. 30 Sekunden.

Zwischen dem Boden des Rührkessels und der Spitze des Rührers wurde in der Mitte des Rührkessels die dispergierte Polymerschmelze aus dem Mischgefäß über eine Leitung (12) entnommen, die über ein Mischventil in einen Separator führte. Die Länge der Leitung 12 zwischen dem dritten Mischgefäß, welches zur Dispergierung der Polymerschmelze in der kontinuierlichen Phase diente, und dem Mischventil betrug 4 m. Im Mischventil wurde die im Mischgefäß erhaltene Emulsion abgekühlt., wobei eine wässrige Phase mit einer Temperatur von 20°C eingesetzt wurde. Nach dem Mischventil wies die Zusammensetzung eine Temperatur von ca. 34°C auf. Die Zuleitung, über die die wässrige Phase mit einer Temperatur von 20°C in das Mischventil eingeleitet wurde, wies einen Durchmesser von ca. 0,012 m auf, wohingegen die Zuleitung, über die die dispergierte Polymerschmelze in das Mischventil eingeleitet wurde, einen Durchmesser von ca. 0,004 m aufwies. Das Geschwindigkeitsverhältnis von Emulsion zu Kühlwasser betrug ca. 6, wobei das Massenverhältnis von Emulsion zu Kühlwasser etwa 1:3 betrug. Diese Werte können über den Druck des Kühlwassers bzw. der Emulsion eingestellt werden, mit der diese Flüssigkeiten in das Mischventil eingeleitet werden.

Die im Mischventil gebildeten Partikel wurden einer Zentrifuge zugeleitet, in der die Wirkstoffpartikel bei 25°C von der kontinuierlichen Phase abgetrennt wurden. Anschließend wurden die Wirkstoffpartikel in einem Vakuumtrockner bei 25°C und 10 mbar für 100 h getrocknet.

Die erhaltenen Partikel zeigten eine Partikelgrößenverteilung von 14,1 $\mu$m < $d_{90,Partikel}$ < 46,3 $\mu$m. Der Ausdruck $d_{90,Partikel}$ bedeutet, dass ca. 90% der Partikel eine Größe im Bereich von 14,1 μm bis 46,3 μm aufwiesen. Die Partikel waren frei von unerwünschten Lösungsmitteln und bestanden aus dem hyperverzweigten fettsäuremodifizierten Polyester und ca. 20 Gew. % Vitamin E, bezogen auf die Partikelmasse.

Beispiel 2

Das Beispiel 1 wurde im Wesentlichen wiederholt, wobei jedoch die Rührgeschwindigkeit des Ultra-Turrax-Rührers zur Herstellung der Dispersion der Polymerschmelze in der wässrigen Phase 9500 Umdrehungen pro Minute betrug.

Die erhaltenen Partikel zeigten eine Partikelgrößenverteilung von 10,5 μm < $d_{90,Partikel}$ < 40,9 μm. Die Partikel waren frei von unerwünschten Lösungsmitteln und bestanden aus dem hyperverzweigten fettsäuremodifizierten Polyester und ca. 20 Gew. % Vitamin E, bezogen auf die Partikelmasse.

Beispiel 3

Das Beispiel 1 wurde im Wesentlichen wiederholt, wobei jedoch die Rührgeschwindigkeit des Ultra-Turrax-Rührers zur Herstellung der Dispersion der Polymerschmelze in der wässrigen Phase 12000 Umdrehungen pro Minute betrug.

Die erhaltenen Partikel zeigten eine Partikelgrößenverteilung von 4,2 μm < $d_{90,Partikel}$ < 32,7 μm. Die Partikel waren frei von unerwünschten Lösungsmitteln und bestanden aus dem hyperverzweigten fettsäuremodifizierten Polyester und ca. 20 Gew. % Vitamin E, bezogen auf die Partikelmasse.

Die Beispiele 1 bis 3 zeigen, dass über Variation der Rührgeschwindigkeit die Partikelgröße eingestellt werden kann.

Beispiel 4

Das Beispiel 2 wurde im Wesentlichen wiederholt, wobei jedoch die Temperatur zum Dispergieren der Polymerschmelze im dritten Mischgefäß (Mischgefäß 6 in Abbildung 1) sowie die Temperatur der kontinuierlichen Phase in Mischgefäß 19 (Abbildung 1) auf 70°C eingestellt wurde. Die Rührgeschwindigkeit des Ultra-Turrax-Rührers zur Herstellung der Dispersion der Polymerschmelze in der wässrigen Phase betrug 9500 Umdrehungen pro Minute.

Die erhaltenen Partikel zeigten eine Partikelgrößenverteilung von 8,6 μm < $d_{90,Partikel}$ < 37,2 μm. Die Partikel waren frei von unerwünschten Lösungsmitteln und bestanden aus dem hyperverzweigten fettsäuremodifiziertem Polyester und ca. 20 Gew. % Vitamin E, bezogen auf die Partikelmasse.

Der Vergleich von Beispiel 2 und Beispiel 4 zeigt, dass die Temperatur, bei der die Dispersion erzeugt wird, einen Einfluss auf die Partikelgröße hat, wobei höhere Temperaturen zu kleineren Partikeln führen.

Beispiel 5

Das Beispiel 2 wurde im Wesentlichen wiederholt, wobei jedoch die Temperatur zum Dispergieren der Polymerschmelze in Mischgefäß 6 sowie die Temperatur der kontinuierlichen Phase in Mischgefäß 19 auf 80°C eingestellt wurde. Die Rührgeschwindigkeit des Ultra-Turrax-Rührers zur Herstellung der Dispersion der Polymerschmelze in der wässrigen Phase betrug 9500 Umdrehungen pro Minute. Weiterhin wurde das Geschwindigkeitsverhältnis von Emulsion zu Kühlwasser auf ca. 3 eingestellt, wobei das Massenverhältnis von Emulsion zu Kühlwasser etwa 2:3 betrug. Diese Werte können über den Druck des Kühlwassers bzw. der Emulsion eingestellt werden, mit der diese Flüssigkeiten in das Mischventil eingeleitet werden.

Die erhaltenen Partikel zeigten eine Partikelgrößenverteilung von 2,8 μm < $d_{90,Partikel}$ < 35,2 μm. Die Partikel waren frei von unerwünschten Lösungsmitteln und bestanden aus dem hyperverzweigten fettsäuremodifizierten Polyester und ca. 20 Gew. % Vitamin E, bezogen auf die Partikelmasse.

Beispiel 6

Das Beispiel 5 wurde im Wesentlichen wiederholt, wobei jedoch die Länge der Leitung zwischen dem Mischgefäß zur Herstellung der Dispersion und dem Mischventil 1 m betrug.

Die erhaltenen Partikel zeigten eine Partikelgrößenverteilung von 1,5 μm < $d_{90,Partikel}$ < 35,1 μm. Die Partikel waren frei von unerwünschten Lösungsmitteln und bestanden aus dem hyperverzweigten fettsäuremodifiziertem Polyester und ca. 20 Gew. % Vitamin E, bezogen auf die Partikelmasse.

Beispiel 7

Das Beispiel 5 wurde im Wesentlichen wiederholt, wobei jedoch die Länge der Leitung zwischen dem Mischgefäß zur Herstellung der Dispersion und dem Mischventil 2 m betrug.

Die erhaltenen Partikel zeigten eine Partikelgrößenverteilung von 2,0 μm < $d_{90,Partikel}$ < 34,9 μm. Die Partikel waren frei von unerwünschten Lösungsmitteln und bestanden aus dem hyperverzweigten fettsäuremodifizierten Polyester und ca. 20 Gew. % Vitamin E, bezogen auf die Partikelmasse.

Beispiel 8

Das Beispiel 5 wurde im Wesentlichen wiederholt, wobei jedoch die Länge der Leitung zwischen dem Mischgefäß zur Herstellung der Dispersion und dem Mischventil 8 m betrug.

Die erhaltenen Partikel zeigten eine Partikelgrößenverteilung von 4,9 μm < $d_{90,Partikel}$ < 35,5 μm. Die Partikel waren frei von unerwünschten Lösungsmitteln

und bestanden aus dem hyperverzweigten fettsäuremodifiziertem Polyester und ca. 20 Gew. % Vitamin E, bezogen auf die Partikelmasse.

Die Beispiele 5 bis 8 zeigen, dass durch die Variation der Länge zwischen Mischgefäß 3 und dem Mischventil überraschend die Partikelgrößenverteilung verbessert werden kann. Hierbei führen längere Strecken, über die die Partikel aggregieren können, zu einer sehr starken Abnahme des Anteils an kleinen Partikeln, wohingegen die Größe der großen Partikel nur unwesentlich zunimmt. Daher können hierdurch überraschend besonders enge Partikelverteilungen auch bei sehr kleinen Partikelgrößen erzielt werden.

## Patentansprüche

1. Verfahren zur Herstellung von Mikropartikeln umfassend die Schritte

    a) Herstellen einer Polymerschmelze umfassend mindestens ein Trägerpolymer und mindestens eine zu verkapselnde Substanz,
    b) Einleiten der Polymerschmelze in eine zweite flüssige Phase, in der das Trägerpolymer zu höchstens 20 Massenprozent löslich ist, wobei die zweite flüssige Phase eine Verfestigungstemperatur unterhalb der Verfestigungstemperatur des Trägerpolymers aufweist,
    c) Dispergieren der Polymerschmelze in der zweiten flüssigen Phase bei einer Temperatur, die größer oder gleich der Verfestigungstemperatur des Trägerpolymers ist und
    d) Verfestigen der in der zweiten flüssigen Phase dispergierten Polymerschmelze durch Abkühlen der zweiten flüssigen Phase auf eine Temperatur unterhalb der Verfestigungstemperatur des Trägerpolymers durch Zuleiten einer Kühlflüssigkeit in einem Mischventil.

2. Verfahren gemäß Anspruch 1, **dadurch gekennzeichnet, dass** das Verfahren kontinuierlich durchgeführt wird.

3. Verfahren gemäß Anspruch 1 oder 2, **dadurch gekennzeichnet, dass** die in der zweiten flüssigen Phase gebildeten Partikel abgetrennt werden.

4. Verfahren gemäß einem der vorhergehenden Ansprüche,
**dadurch gekennzeichnet, dass** die zweite flüssige Phase eine hydrophile Phase ist.

5. Verfahren gemäß einem der vorhergehenden Ansprüche,
**dadurch gekennzeichnet, dass** mindestens 80 Gew.-% der erhaltenen Partikel innerhalb eines Größenbereichs von höchstens 100 μm liegen.

6. Verfahren gemäß einem der vorhergehenden Ansprüche,
**dadurch gekennzeichnet, dass** das Volumenverhältnis von Kühlflüssigkeit zu dem Produkt aus Schritt c) im Bereich von 10:1 bis 1:1 liegt.

7. Verfahren gemäß einem der vorhergehenden Ansprüche,
**dadurch gekennzeichnet, dass** das Verhältnis der Strömungsgeschwindigkeit, mit der das Produkt aus Schritt c) in das Mischventil eingeleitet wird, zur Strömungsgeschwindigkeit der Kühlflüssigkeit größer als 1 ist.

8. Verfahren gemäß einem der vorhergehenden Ansprüche,
**dadurch gekennzeichnet, dass** zwischen dem Dispergieren der Polymerschmelze und dem Verfestigen eine Verweilzeit von 0,5 Sekunden bis 1 Stunde liegt.

9. Anlage zur Durchführung des Verfahrens gemäß einem der Ansprüche 1 bis 8, umfassend mindestens drei Mischgefäße (3, 6, 19), einen Separator (13), eine Zuführung (12) und ein Mischventil (26), wobei mindestens zwei Mischgefäße (3, 19) über Zuführungen (5, 9) mit einem dritten Mischgefäß (6) verbunden sind, das dritte Mischgefäß (6) mit dem Separator (13) verbunden ist und das Mischventil in der Zuführung (12) zwischen dem Mischgefäß (6) und dem Separator (13) vorgesehen ist.

10. Anlage gemäß Anspruch 9, **dadurch gekennzeichnet, dass** die Anlage mindestens einen Trockner (17) aufweist, der mit dem Separator (13) verbunden ist.

11. Anlage gemäß Anspruch 9 oder 10, **dadurch gekennzeichnet, dass** die Anlage eine Vorrichtung zum Waschen von Partikeln aufweist.

## Claims

1. Process for preparing microparticles, comprising the steps of

    a) preparing a polymer melt comprising at least one carrier polymer and at least one substance to be encapsulated,
    b) introducing the polymer melt into a second liquid phase in which the carrier polymer is soluble to an extent of at most 20 per cent by mass, the second liquid phase having a solidification temperature below the solidification temperature of the carrier polymer,
    c) dispersing the polymer melt in the second liquid phase at a temperature which is greater than

or equal to the solidification temperature of the carrier polymer and

d) solidifying the polymer melt dispersed in the second liquid phase by cooling the second liquid phase to a temperature below the solidification temperature of the carrier polymer by supplying a cooling liquid in a mixing valve.

2. Process according to Claim 1, **characterized in that** the process is performed continuously.

3. Process according to Claim 1 or 2, **characterized in that** the particles formed in the second liquid phase are separated.

4. Process according to any of the preceding claims, **characterized in that** the second liquid phase is a hydrophilic phase.

5. Process according to any of the preceding claims, **characterized in that** at least 80% by weight of the resulting particles are within a size range of at most 100 $\mu$m.

6. Process according to any of the preceding claims, **characterized in that** the volume ratio of cooling liquid to the product from step c) is in the range from 10:1 to 1:1.

7. Process according to any of the preceding claims, **characterized in that** the ratio of the flow rate with which the product from step c) is introduced into the mixing valve to the flow rate of the cooling liquid is greater than 1.

8. Process according to any of the preceding claims, **characterized in that** there is a residence time of 0.5 second to 1 hour between the dispersing of the polymer melt and the solidification.

9. Unit for performing the process according to any of Claims 1 to 8, comprising at least three mixing vessels (3, 6, 19), a separator (13), a feed line (12) and a mixing valve (26), at least two of the mixing vessels (3, 19) being connected to a third mixing vessel (6) via feed lines (5, 9), the third mixing vessel (6) being connected to the separator (13), and the mixing valve being provided in the feed line (12) between the mixing vessel (6) and the separator (13).

10. Unit according to Claim 9, **characterized in that** the unit comprises at least one dryer (17) which is connected to the separator (13).

11. System according to Claim 9 or 10, **characterized in that** the unit comprises a device for washing particles.

**Revendications**

1. Procédé pour la production de microparticules, comprenant les étapes

a) préparation d'une masse fondue de polymère comprenant au moins un polymère de support et au moins une substance à encapsuler,

b) introduction de la masse fondue de polymère dans une deuxième phase liquide, dans laquelle le polymère de support est soluble à raison d'au maximum 20 % en masse, la deuxième phase liquide ayant une température de solidification inférieure à la température de solidification du polymère de support,

c) dispersion de la masse fondue de polymère dans la deuxième phase liquide à une température qui est supérieure ou égale à la température de solidification du polymère de support et

d) solidification de la masse fondue de polymère dispersée dans la deuxième phase liquide, par refroidissement de la deuxième phase liquide à une température inférieure à la température de solidification du polymère de support, par introduction d'un liquide de refroidissement dans une vanne mélangeuse.

2. Procédé selon la revendication 1, **caractérisé en ce que** le procédé est effectué en continu.

3. Procédé selon la revendication 1 ou 2, **caractérisé en ce que** les particules formées dans la deuxième phase liquide sont séparées.

4. Procédé selon l'une quelconque des revendications précédentes, **caractérisé en ce que** la deuxième phase liquide est une phase hydrophile.

5. Procédé selon l'une quelconque des revendications précédentes, **caractérisé en ce qu'**au moins 80 % en poids des particules obtenues se trouvent à l'intérieur d'une plage de taille d'au maximum 100 $\mu$m.

6. Procédé selon l'une quelconque des revendications précédentes, **caractérisé en ce que** le rapport volumique du liquide de refroidissement au produit provenant de l'étape c) se situe dans la plage de 10:1 à 1:1.

7. Procédé selon l'une quelconque des revendications précédentes, **caractérisé en ce que** le rapport de la vitesse d'écoulement à laquelle le produit provenant de l'étape c) est introduit dans la vanne de mélange, à la vitesse d'écoulement du liquide de refroidissement, est supérieur à 1.

8. Procédé selon l'une quelconque des revendications précédentes, **caractérisé en ce qu'**un temps de séjour de 0,5 seconde à 1 heure existe entre la dispersion de la masse fondue de polymère et la solidification.

**9.** Installation pour la mise en oeuvre du procédé selon l'une quelconque des revendications 1 à 8, comprenant au moins trois récipients de mélange (3, 6, 19), un séparateur (13), un conduit d'alimentation (12) et une vanne de mélange (26), au moins deux récipients de mélange (3, 19) étant reliés par des conduits d'alimentation (5, 9) à un troisième récipient de mélange (6), le troisième récipient de mélange (6) étant relié au séparateur (13) et la vanne de mélange dans le conduit d'alimentation (12) étant prévue entre le récipient de mélange (6) et le séparateur (13).

**10.** Installation selon la revendication 9, **caractérisée en ce que** l'installation comporte au moins un sécheur (17) qui est relié au séparateur (13).

**11.** Installation selon la revendication 9 ou 10, **caractérisée en ce que** l'installation comporte un dispositif destiné au lavage de particules.

Figur 1

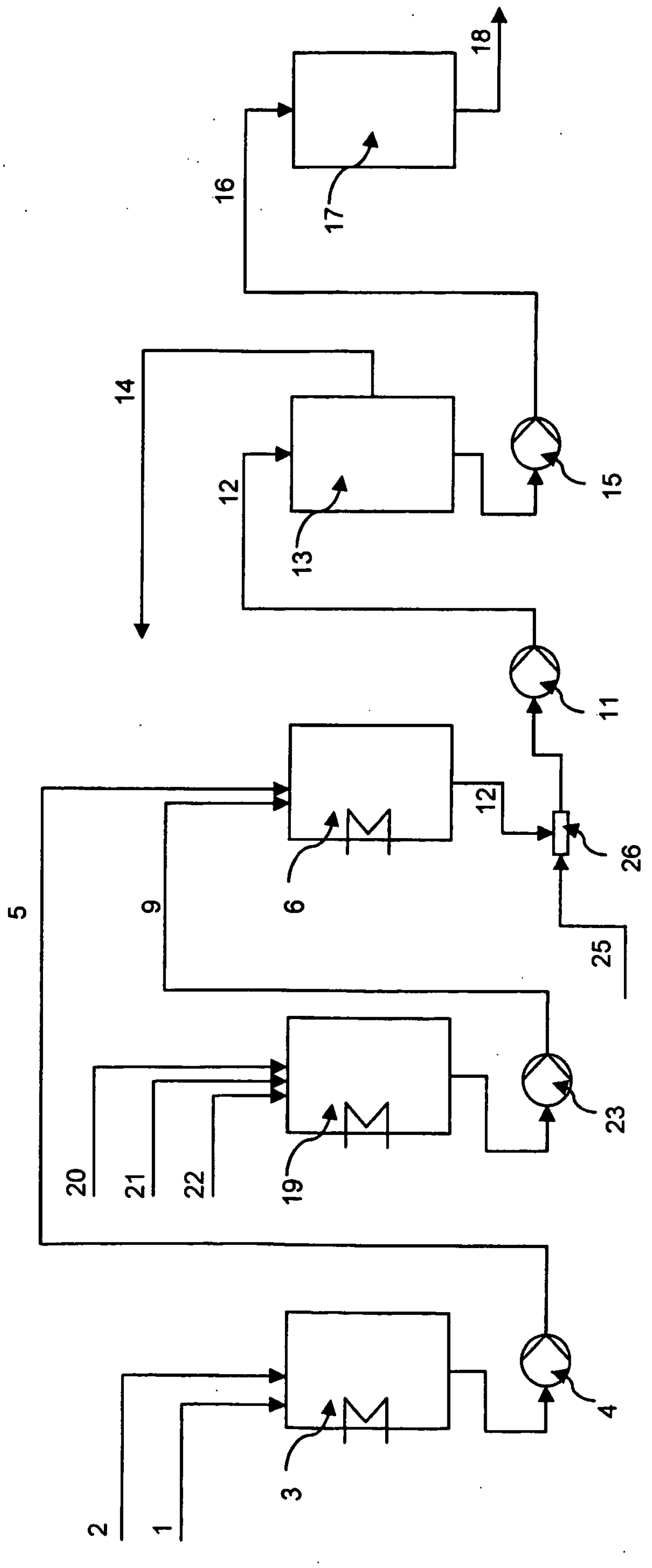

Figur 1a

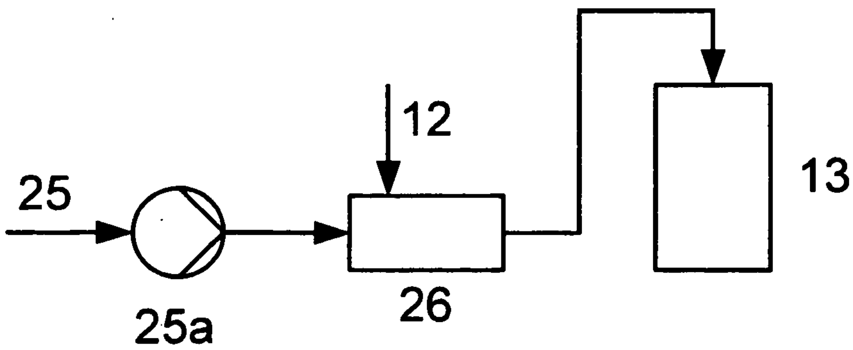


Figur 1b

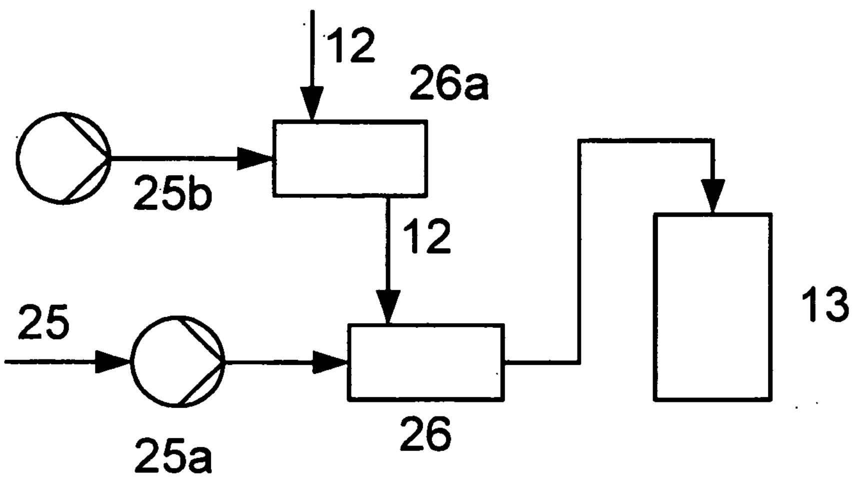

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3916020
- WO 9715389 A
- WO 05072702 A
- WO 04064752 A
- DE 10061932
- WO 0072830 A
- EP 914095 A
- WO 9742940 A
- EP 0630389 A
- WO 9706825 A
- US 5041516 A
- US 5136014 A
- US 5183862 A
- US 5196502 A
- US 5225522 A
- US 5227462 A
- US 5362843 A
- US 5418301 A
- WO 9830604 A
- WO 2004072153 A
- WO 00065024 A
- WO 2005034909 A
- WO 03037383 A
- WO 0006267 A
- WO 03033027 A
- WO 0056804 A1
- WO 9318079 A
- WO 9317060 A
- EP 869984 A
- WO 0059982 A
- EP 630389 A
- EP 0630389 B1
- WO 9301760 A


### In der Beschreibung aufgeführte Nicht-Patentliteratur


- **Gamse et al.** *Chemie Ingenieur Technik,* 2005, vol. 77 (6), 669-679
- **von Gamse et al.** *Chemie Ingenieur Technik,* 2005, vol. 77 (6), 669-679
- **Tom, J.W. ; Lim, G.B. ; Debendetti, P.G. ; Prod'homme, R.K.** Supercritical Fluid Engineering Science. American Chemical Society, 1993
- **Pérez de Diego, Y.** *Production of Controlled Drug Delivery Microparticles using Supercritical CO2,* 2004
- **Shariati, A. ; Peters, C.J.** Recent developments in particle design using supercritical fluids. *Current Opinion in Solid State & Materials Science,* 2003, vol. 7 (4-5), 371-383
- **R. Arshady.** Microspheres and microcapsules - A survey of manufacturing techniques, Part III: Coacervation. *Polymer Engineering and Science,* 1990, vol. 30, 905ff
- **R. Arshady.** Microspheres and microcapsules - A survey of manufacturing techniques, Part III: Coacervation. *Polymer Engineering and Science,* 1990, vol. 30, 905ff
- **Jain, R.A.** The manufacturing techniques of various drug loaded biodegradable poly(lactide-co-glycolide) (PLGA) devices. *Biomaterials,* 2000, vol. 21, 2475-2490
- **Jung, J. ; Perrut, M.** Particle design using supercritical fluids: Literature and patent survey. *Journal of Supercritical Fluids,* 2001, vol. 20 (3), 179-219
- **Subramaniam, B. ; Rajewski, R.A. ; Snavely, K.** Pharmaceutical processing with supercritical carbon dioxide. *Journal of Pharmaceutical Sciences,* 1997, vol. 86 (8), 885-890
- **C. M. Adeyeye ; J. C. Price.** *Pharmaceutical Research,* 1991, vol. 8 (11), 1377-1383
- **A. Paradkar et al.** *AAPS PharmSciTech,* 2003, vol. 4 (4
- **A. Raziel.** Wax Microemboli Tailored for Therapeutic Embolization. *AJR,* Februar 1981, vol. 134, 404-405
- **S. Benita et al.** *Journal of Pharmaceutical Sciences,* September 1986, vol. 75 (9), 847-851
- **N. Mani.** *Drug Development and Industrial Pharmacy,* 2004, vol. 30 (1), 83-93
- **N. Mani et al.** *J. Microencapsulation,* 2004, vol. 21 (2), 125-135
- **E. Mathiowitz ; R. Langer.** *Journal of Controlled Release,* 1987, vol. 5, 13-22
- **Fréchet J.M.J. ; Tomalia D.A.** Dendrimers and Other Dendritic Polymers. John Wiley &Sons, Ltd, 2001
- **Jikei M. ; Kakimoto M.** Hyperbranched polymers: a promising new class of materials. *Prog. Polym. Sci.,* 2001, vol. 26, 1233-1285

- **Gao C. ; Yan D.** Hyperbranched Polymers: from synthesis to applications. *Prog. Polym. Sci.,* 2004, vol. 29, 183-275
- *J. Chem. Soc. Perkin Trans.,* 1992, 2459-2469
- *Macromolecules,* 1993, vol. 26, 4617-4623
- *Macromol. Biosci.,* 2005, vol. 5, 662-668
- **Burgath.** *Macromol.Chem. Phys.,* 2000, vol. 201, 782-791
- **D.Hölter ; A.Burgath ; H.Frey.** *Acta Polymer,* 1997, vol. 48, 30
- **H. Magnusson ; E. Malmström ; A. Hult ; M. Joansson.** *Polymer,* 2002, vol. 43, 301
- Römpp Chemie Lexikon
- Ullmann's Encyclopedia of Industrial Chemistry
- **K. Mohanty ; M. Misra ; G. Hinrichsen.** *Macromolecular Materials and Engineering,* 2000, vol. 276-277 (1), 1-24
- **Dubbel.** Taschenbuch für den Maschinenbau. Springer-Verlag, 1997